# EUROPEAN PATENT SPECIFICATION

(11) **EP 1 560 526 B1**
(45) Date of publication and mention of the grant of the patent: **06.06.2007**
(21) Application number: 03786565.6
(22) Date of filing: 12.11.2003
(51) Int. Cl.: A61B 17/00, A61B 17/04

(54) **DEVICES FOR HEART VALVE TREATMENT**
VORRICHTUNGEN ZUR HERZKLAPPENBEHANDELUNG
DISPOSITIFS DE TRAITEMENT DE VALVULES CARDIAQUES

(30) Priority: 12.11.2002 US 425519 P; 10.11.2003 US 704143; 10.11.2003 US 704145
(43) Date of publication of application: 10.08.2005
(73) Proprietor: Myocor, Inc., Maple Grove, NW 55311 (US)
(72) Inventor: VIDLUND, Robert, M., Maplewood, MN 55109 (US); KALGREEN, Jason, E., Plymouth, MN 55446 (US); MORTIER, Todd, J., Minneapolis, MN 55408 (US); SCHWEICH, Cyril, J., Jr., Maple Grove, MN 55311 (US); SCHROEDER, Richard, Fridley, MN 55432 (US); KUSZ, David, Minneapolis, MN 55406 (US); EKVALL, Craig, A., Elk River, MN 55330 (US); MATTHEES, Edward, Minneapolis, MN 55414 (US)
(74) Representative: Molnia, David
(86) International application number: PCT/US2003/035037
(87) International publication number: WO 2004/043265

(56) References cited:
- WO-A-02/30292
- US-A1- 2002 161 275
- US-B1- 6 183 411
- US-B1- 6 406 420

## Description

### Field of the Invention

The present invention relates to devices for treating and improving the performance of dysfunctional heart valves. More particularly, the invention relates to devices that passively assist to reshape a dysfunctional heart valve to improve its performance.

### Background of the Invention

Various etiologies may result in heart valve insufficiency depending upon both the particular valve as well as the underlying disease state of the patient For instance, a congenital defect may be present resulting in poor coaptation of the valve leaflets, such as in the case of a monocusp aortic valve, for example. Valve insufficiency also may result from an infection, such as rheumatic fever, for example, which may cause a degradation of the valve leaflets. Functional regurgitation also may be present. In such cases, the valve components may be normal pathologically, yet may be unable to function properly due to changes in the surrounding environment. Examples of such changes include geometric alterations of one or more heart chambers and/or decreases in myocardial contractility. In any case, the resultant volume overload that exists as a result of an insufficient valve may increase chamber wall stress. Such an increase in stress n ay eventually result in a dilatory process that further exacerbates valve dysfunction and degrades cardiac efficiency.

Mitral valve regurgitation often may be driven by the functional changes described above. Alterations in the geometric relationship between valvular components may occur for numerous reasons, including events ranging from focal myocardial infarction to global ischemia of the myocardial tissue. Idiopathic dilated cardiomyopathy also may drive the evolution of functional mitral regurgitation. These disease states often lead to dilatation of the left ventricle. Such dilatation may cause papillary muscle displacement and/or dilatation of the valve annulus. As the papillary muscles move away from the valve annulus, the chordae connecting the muscles to the leaflets may become tethered. Such tethering may restrict the leaflets from closing together, either symmetrically or asymmetrically, depending on the relative degree of displacement between the papillary muscles. Moreover, as the annulus dilates in response to chamber enlargement and increased wall stress, increases in annular area and changes in annular shape may increase the degree of valve insufficiency. Annular dilatation is typically concentrated on the posterior aspect, since this aspect is directly associated with the dilating left ventricular free wall and not directly attached to the fibrous skeleton of the heart. Annular dilatation also may result in a flattening of the valve annulus from its normal saddle shape.

Alterations in functional capacity also may cause valve insufficiency. In a normally functioning heart, the mitral valve annulus contracts during systole to assist in leaflet coaptation. Reductions in annular contractility commonly observed in ischemic or idiopathic cardiomyopathy patients therefore hamper the closure of the valve. Further, in a normal heart, the papillary muscles contract during the heart cycle to assist in maintaining proper valve function. Reductions in or failure of the papillary muscle function also may contribute to valve regurgitation. This may be caused by infarction at or near the papillary muscle, ischemia, or other causes, such as idiopathic dilated cardiomyopathy, for example.

The degree of valve regurgitation may vary, especially in the case of functional insufficiency. In earlier stages of the disease, the valve may be able to compensate for geometric and/or functional changes in a resting state. However, under higher loading resulting from an increase in output requirement, the valve may become incompetent. Such incompetence may only appear during intense exercise, or alternatively may be induced by far less of an exertion, such as walking up a flight of stairs, for example.

Conventional techniques for managing mitral valve dysfunction include either surgical repair or replacement of the valve or medical management of the patient. Medical management typically applies only to early stages of mitral valve dysfunction, during which levels of regurgitation are relatively low. Such medical management tends to focus on volume reductions, such as diuresis, for example, or afterload reducers, such as vasodilators, for example.

Early attempts to surgically treat mitral valve dysfunction focused on replacement technologies. In many of these cases, the importance of preserving the native subvalvular apparatus was not fully appreciated and many patients often acquired ventricular dysfunction or failure following the surgery. Though later experience was more successful, significant limitations to valve replacement still exist. For instance, in the case of mechanical prostheses, lifelong therapy with powerful anticoagulants may be required to mitigate the thromboembolic potential of these devices. In the case of biologically derived devices, in particular those used as mitral valve replacements, the long-term durability may be limited. Mineralization induced valve failure is common within ten years, even in younger patients. Thus, the use of such devices in younger patient groups is impractical.

Another commonly employed repair technique involves the use of annuloplasty rings. These rings originally were used to stabilize a complex valve repair. Now, they are more often used alone to improve mitral valve function. An annuloplasty ring has a diameter that is less than the diameter of the enlarged valve annulus. The ring is placed in the valve annulus and the tissue of the annulus sewn or otherwise secured to the ring. This causes a reduction in the annular circumference and an increase in the leaflet coaptation area. Such rings, however, generally flatten the natural saddle shape of the valve and hinder the natural contractility of the valve annulus. This may be true even when the rings have relatively high flexibility.

To further reduce the limitations of the therapies described above, purely surgical techniques for treating valve dysfunction have evolved. Among these surgical techniques is the Alfiere stitch or so-called bowtie repair. In this surgery, a suture is placed substantially centrally across the valve orifice joining the posterior and anterior leaflets to create leaflet apposition. Another surgical technique includes plication of the posterior annular space to reduce the cross-sectional area of the valve annulus. A limitation of each of these techniques is that they typically require opening the heart to gain direct access to the valve and the valve annulus. This generally necessitates the use of cardiopulmonary bypass, which may introduce additional morbidity and mortality to the surgical procedures. Additionally, for each of these procedures, it is very difficult to evaluate the efficacy of the repair prior to the conclusion of the operation.
WO 02/30292 discloses a device for improving heart function according to the preamble of claim 1.

Due to these drawbacks, devising effective techniques that could improve valve function without the need for cardiopulmonary bypass and without requiring major remodeling of the valve may be advantageous. In particular, passive techniques to change the shape of the heart chamber and/or associated valve and reduce regurgitation while maintaining substantially normal leaflet motion may be desirable. Further, advantages may be obtained by a technique that reduces the overall time a patient is in surgery and under the influence of anesthesia. It also may be desirable to provide a technique for treating valve insufficiency that reduces the risk of bleeding associated with anticoagulation requirements of cardiopulmonary bypass. In addition, a technique that can be employed on a beating heart would allow the practitioner an opportunity to assess the efficacy of the treatment and potentially address any inadequacies without the need for additional bypass support.

### Summary of the Invention

To address these needs, the present invention provides non-limiting embodiments, devices for improving the function of a valve (e.g., mitral valve) by positioning an implantable device outside and adjacent the heart wall such that the device alters the shape of the heart wall acting on the valve. The implantable device may include two anchor ends with a interconnecting member connected therebetween. The anchor ends and the interconnecting member may be positioned on the outside of the heart. A protrusion may be connected to the interconnecting member between the anchor ends. The anchor ends may be connected to the heart wall around the dysfunctional valve, and the interconnecting member may be tightened or cinched therebetween. Because the heart wall is generally curved, the act of cinching the interconnecting member between the attached anchor ends may cause the interconnecting member to apply an inward force against the heart wall acting on the dysfunctional valve, and/or may shorten the distance between the anchor ends and thus deform the heart wall inward to act on the dysfunctional valve. The inward force may act on any one of or any combination of valve structures (e.g., valve annulus, papillary muscles, etc.) and/or adjacent anatomical coronary structures. If a protrusion is utilized, it may be used to apply and focus additional force against the heart wall.

### Brief Description of the Drawings

Besides the structural and procedural arrangements set forth above, the invention could include a number of other arrangements, such as those explained hereinafter. It is to be understood that both the foregoing description and the following description are exemplary. The accompanying drawings are included to provide a further understanding of the invention and are incorporated in and constitute a part of this specification. The drawings illustrate exemplary embodiments of the invention and, together with the description, serve to explain certain principles. In the drawings,

Figures 1A and 1B are bottom and side views, respectively, of an exemplary, non-limiting embodiment of an implantable device utilizing a protrusion;

Figures 1C and 1D are bottom and side views, respectively, of an exemplary, non-limiting alternative embodiment of an implantable device without a protrusion;

Figures 2A - 2C are sectional views of a patient's trunk at the level of the mitral valve of the heart, showing an example of where the implantable devices may be positioned in the short axis view, and showing the effects of the implantable devices on mitral valve function;

Figure 3 is a sectional view of a patient's heart bisecting the mitral valve, showing an example of where the implantable devices may be positioned in the long axis view;

Figure 4 is an angiographic illustration of a patient's heart, showing an example of where the implantable devices may be positioned relative to the coronary arteries;

Figures 5A - 5D are perspective views of more specific embodiments of implantable devices of the present invention;

Figure 5E is a schematic illustration of a cable locking mechanism for use in any of the implantable devices shown in Figures 5A - 5D;

Figure 6A is a perspective plan view of a delivery system for implanting the implantable devices shown in Figures 5A - 5D;

Figure 6B is a perspective bottom view of an anchor catheter for use in the delivery system shown in Figure 6A;

Figure 7 is a perspective plan view of an alternative delivery system for implanting the implantable devices shown in Figures 5A - 5D;

Figure 8 is a perspective view of a sizing device for use in adjusting the implantable devices shown in Figures 5A - 5D;

Figure 9 is a perspective exploded view of an access system to facilitate pericardial access of the delivery systems;

Figure 10 is a partially sectioned side view of a distal portion of the access device shown in Figure 9, illustrating engagement with the pericardial sac;

Figure 11 is an illustration schematically showing a pericardial access approach for delivery of the implantable devices;

Figure 12 is a schematic plan view of a catheter and guide wire for use in delivering implantable devices by transluminal techniques;

Figures 13A and 13B are cross sectional views of the catheter shown in Figure 12 taken along line 13 - 13;

Figure 14 is a cross sectional view of the catheter shown in Figure 12 taken along line 14 -14;

Figures 15A and 15B are schematic top and side views of a transdermal access port connected to an implantable device by a flexible tube;

Figure 16 is a schematic plan view of a guide catheter for use in delivering implantable devices by transluminal techniques;

Figure 17 is a schematic plan view of an isolation catheter for use in delivering implantable devices by transluminal techniques;

Figures 18 - 30 are schematic illustrations of various design alternatives of implantable devices; and

Figures 31A and 31B are schematic views of a catheter for use in delivering implantable devices by transthoracic techniques.

### Detailed Description of Exemplary Embodiments

The following detailed description should be read with reference to the drawings in which similar elements in different drawings are numbered the same. The drawings, which are not necessarily to scale, depict illustrative embodiments and are not intended to limit the scope of the invention.

The various aspects of the devices described herein generally pertain to devices for treating heart conditions, including, for example, dilatation, valve incompetencies, including mitral valve leakage, and other similar heart failure conditions. Each disclosed device may operate passively in that, once placed on the heart, it does not require an active stimulus, either mechanical, electrical, hydraulic, pneumatic, or otherwise, to function. Implanting one or more of the devices operates to assist in the apposition of heart valve leaflets to improve valve function.

In addition, these devices may either be placed in conjunction with other devices that, or may themselves function to, alter the shape or geometry of the heart, locally and/or globally, and thereby further increase the heart's efficiency. That is, the heart experiences an increased pumping efficiency through an alteration in its shape or geometry and concomitant reduction in stress on the heart walls, and through an improvement in valve function.

However, the devices disclosed herein for improving valve function can be "stand-alone" devices, that is, they do not necessarily have to be used in conjunction with additional devices for changing the shape of a heart chamber or otherwise reducing heart wall stress. It also is contemplated that a device for improving valve function may be placed relative to the heart without altering the shape of the chamber, and only altering the shape of the valve itself. In other words, the devices described herein involve geometric reshaping of portions of the heart and treating valve incompetencies.

The devices described herein offer numerous advantages over the existing treatments for various heart conditions, including valve incompetencies. The devices are relatively easy to manufacture and use, and the transluminal, transthoracic, and surgical techniques and tools for implanting the devices do not require the invasive procedures of current surgical techniques. For instance, these techniques do not require removing portions of the heart tissue, nor do they necessarily require opening the heart chamber or stopping the heart during operation. For these reasons, the techniques for implanting the devices disclosed herein also are less risky to the patient than other techniques. The less invasive nature of these techniques and tools may also allow for earlier intervention in patients with heart failure and/or valve incompetencies.

Although the devices, are discussed hereinafter in connection with their use for the mitral valve of the heart, these devices may be used for other valves of the heart for similar purposes. One of ordinary skill in the art would understand that the use of the devices described herein also, could be employed for other valves of the heart. The mitral valve has been selected for illustrative purposes because a large number of the disorders occur in connection with the mitral valve.

The devices described herein are discussed herein with reference to the human heart H, but may be equally applied to other animal hearts not specifically mentioned herein. For purposes of discussion and illustration, several anatomical features may be labeled as follows: left ventricle LV; right ventricle RV; left atrium LA; ventricular septum VS; right ventricular free wall RVFW; left ventricular free wall LVFW; atrioventricular groove AVG; mitral valve MV; tricuspid valve TV; aortic valve AV; pulmonary valve PV; papillary muscle PM; chordae tendeneae CT (or simply chordae); anterior leaflet AL; posterior leaflet PL; coaptation line CL; annulus AN; ascending aorta AA; thoracic aorta TA; azygos vein AZV; coronary sinus CS; cardiac vein CV; right coronary artery RCA; left anterior descending artery LAD; obtuse marginal artery OM; circumflex artery CFX; left lung LL; right lung RL; dermal layer DL; sternum ST; xiphoid XPH; diaphragm DPH; and vertebrae VRT.

**General Description of Exemplary Implant Devices**

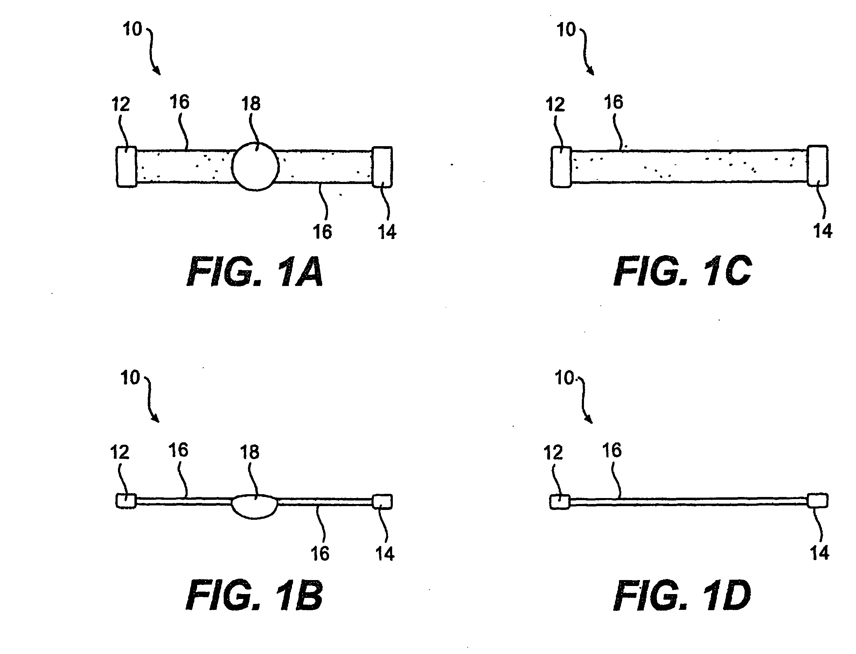

With reference to Figures 1A and 1B, a generic implantable device 10 is shown schematically. The implantable device 10 may generally include two or more anchor ends 12/14 with a interconnecting member 16 connected therebetween. The anchor ends 12/14 may be configured to permanently or releasably attach to the outside of the heart wall. The interconnecting member 16 may be selectively tightened or loosened to correspondingly affect the tension between the anchor ends 12/14. A protrusion 18 may be connected to the interconnecting member 16 between the anchor ends 12/14. Alternatively, as shown in Figures 1C and 1D, the implantable device 10 may utilize anchor ends 12/14 and interconnecting member 16 alone, without the use of a protrusion 18. With or without protrusion 18, the interconnecting member may be generally flexible to conform to the outer surface of the heart. Protrusion 18 may alternatively be referred to as a space filling member or a focal member. Interconnecting member 16 may alternatively be referred to as an elongate member or as a tension member.

The position of the protrusion 18 may be adjusted relative to the anchor ends 12/14. To accommodate such adjustment, the interconnecting member 16 may be fixedly connected to one or both of the anchor ends 12/14 and adjustably connected to the protrusion 18. Alternatively, the interconnecting member 16 may be fixedly connected to the protrusion 18 and adjustably connected to one or both of the anchor ends 12/14. In both instances, the length of the interconnecting member 16 between the protrusion 18 and the anchor ends 12/14 may be adjusted to change the position of the protrusion 18 relative to the anchor ends 12/14.

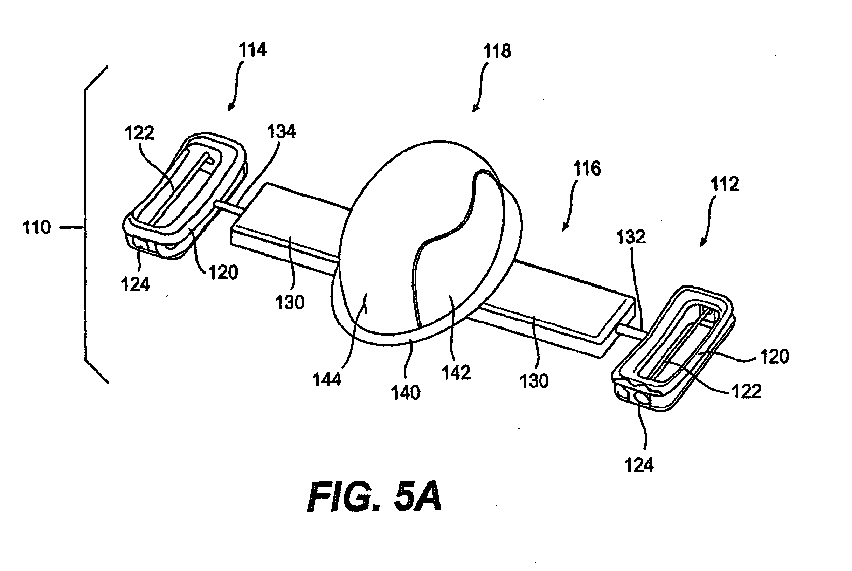

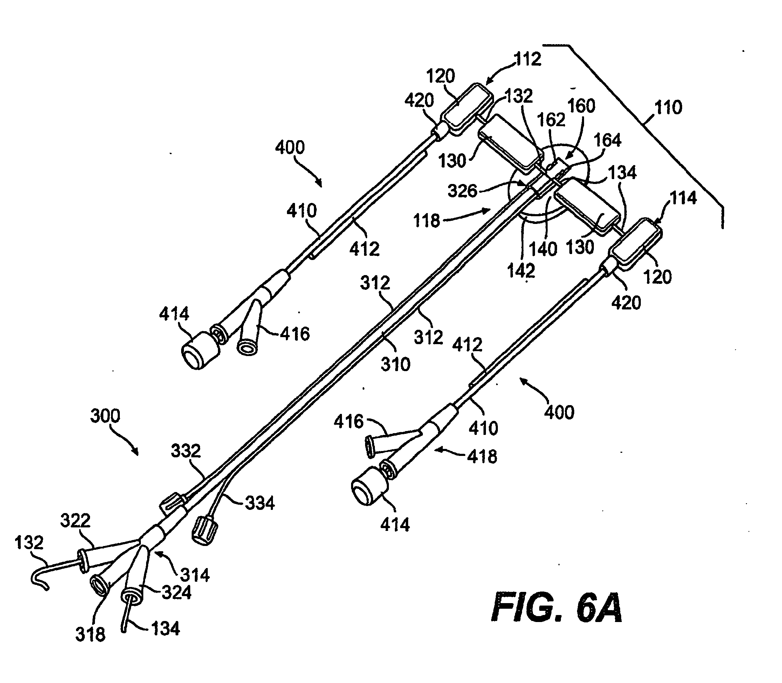

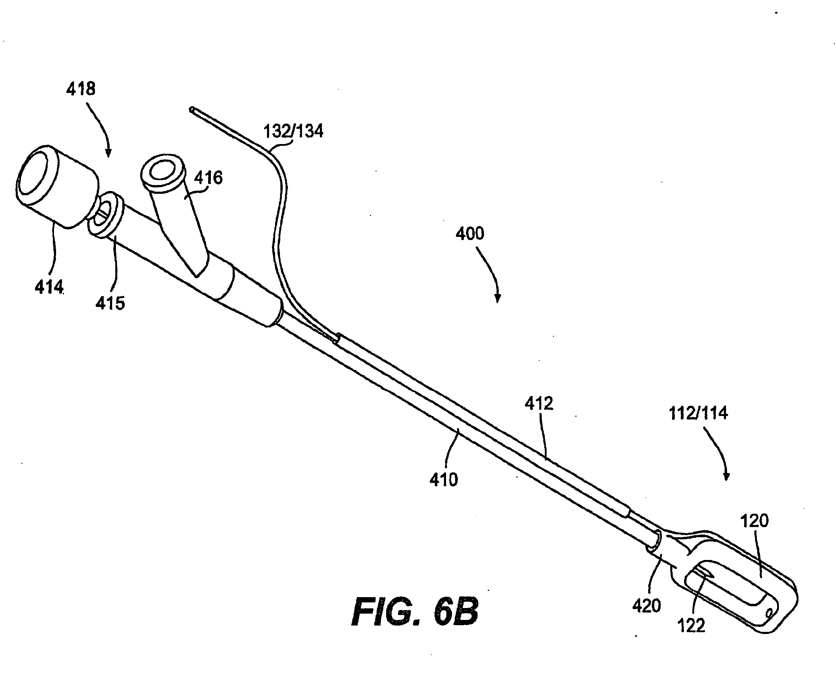

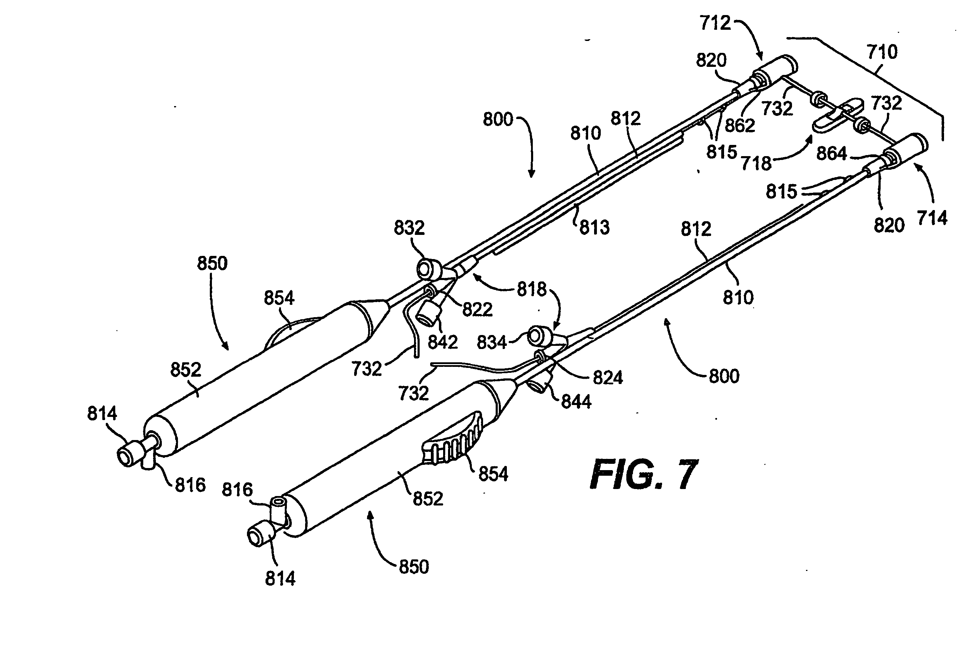

The anchors 12/14 serve to secure the ends of the interconnecting member 16 to the heart wall. The anchors 12/14 may comprise vacuum cups with tissue piercing pins for securement as described in more detail with reference to Figures 5A - 5D. The anchors 12/14 may be remotely activated as described with reference to Figures 6 and 7. The anchors 12/14 may selectively connect to some tissue (e.g., epicardium, myocardium) while remaining free of other tissue (e.g. pericardium). Various alternative anchor embodiments are envisioned, such as tines, screws, sutures, adhesives, etc., and/or a tissue in-growth promoting material (e.g., Dacron fabric). For example, the anchors 12/14 may comprise tines that extend through the epicardium and into the myocardium, and optionally extend through the endocardium into a heart chamber. Additional alternative anchor embodiments are described by Vidlund et al., '519.

The interconnecting member 16 may be fixed or selectively fixed (i.e., adjustable) to each of the anchors 12/14 and/or the protrusion 18 as described above. The interconnecting member may be made fixed or adjustable using, for example, a lock pin technique as described in more detail with reference to Figures 5A - 5D.

In conjunction with interconnecting member 16, pericardial tissue may be used to connect the anchor ends 12/14 and protrusion 18 (if used). For example, a first anchor end 12 may be fixedly secured to both the epicardium and the pericardium using an anchor device with open top and bottom surfaces as described in Vidlund et al., '519. The second anchor end 14 may be secured to epicardium, and the protrusion 18 may be secured to the pericardium (by using an anchor device for the protrusion, 18). The interconnecting member 16 may be fixedly connected to the protrusion 18 and adjustably connected to the second anchor end 14 (or visa-versa) such that the position of the protrusion 18 may be adjusted (e.g., cinched) relative to the second anchor end 14. By virtue of the common pericardial connection between the first anchor 12 and the protrusion 18, cinching the interconnecting member 16 between the protrusion 18 and the second anchor 14 also causes cinching between the protrusion 18 and the first anchor 12, without requiring the interconnecting, member 16 to be connected to the first anchor 12.

The interconnecting member 16 may be elongate and will normally be in tension when implanted. The interconnecting member may comprise a flexible and biocompatible multifilament braid in the form of a string or strap, for example. If a string or chord is used, for example, an atraumatic pad (as seen in Figure 5A) may be disposed on the interconnecting member 16 to avoid stress concentration on the heart wall by the interconnecting member 16.

The interconnecting member 16 may be formed as described in U.S. Patent No. 6,537,198 to Vidlund et al.. In particular, the interconnecting member 16 may comprise a composite structure including an inner cable to provide mechanical integrity and an outer covering to provide biocompatibility The inner cable of interconnecting member 16 may have a multifilament braided-cable of high performance polymeric fibers such as ultra high molecular weight polyethylene available under the trade names Spectra^{™} and Dyneema^{™}, polyester available under the trade name Dacron^{™}, or liquid crystal polymer available under the trade name Vectran^{™}. The filaments forming the inner cable may be combined, for example, in yarn bundles of approximately 50 individual filaments, with each yarn bundle being approximately 180 denier, and two bundles may be paired together (referred to as 2-ply) and braided with approximately 16 total bundle pairs with approximately 20 to 50 picks per inch (number of linear yarn overlaps per inch).

The outer covering surrounding the inner cable of the interconnecting member 16 may provide properties that facilitate sustained implantation, and may thus be formed of a material that is biocompatible and allows for tissue ingrowth. For example, the outer covering surrounding the inner cable of the interconnecting member 16 may be made of a polyester material such as Dacron or ePTFB. If an atraumatic pad is used, it may be formed of, coated with, or covered by the same or similar material as the outer covering of the interconnecting member to promote tissue in-growth for additional anchoring effect. For example, the atraumatic pad may be formed of ePTFE which is biocompatible, promotes tissue in-growth, and conserves cross-sectional size and shape despite elongation.

The protrusion 18 may comprise a balloon, plug, or other mechanical spacer or structure, and may be fixedly or adjustably connected to the interconnecting member 16. The protrusion 18 may be centered between the anchors 12/14, or may be eccentrically positioned therebetween. One or more protrusions 18 may be used, and the protrusions may have various geometries depending on the desired allocation of forces acting on the heart wall. The protrusion 18 may be coated or covered by a tissue in-growth promoting material to secure the protrusion to the heart wall in the desired position, and the material may be highly elastic or otherwise stretchable to permit expansion of the protrusion 18. Examples of suitable materials include ePTFE and polyester knits.

**Description of Exemplary Implant Positions and Functions**

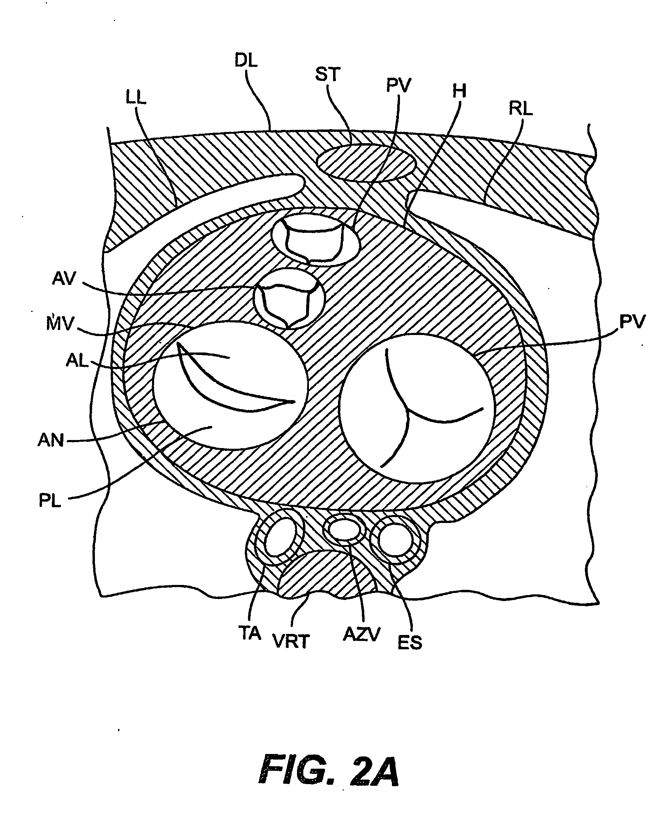

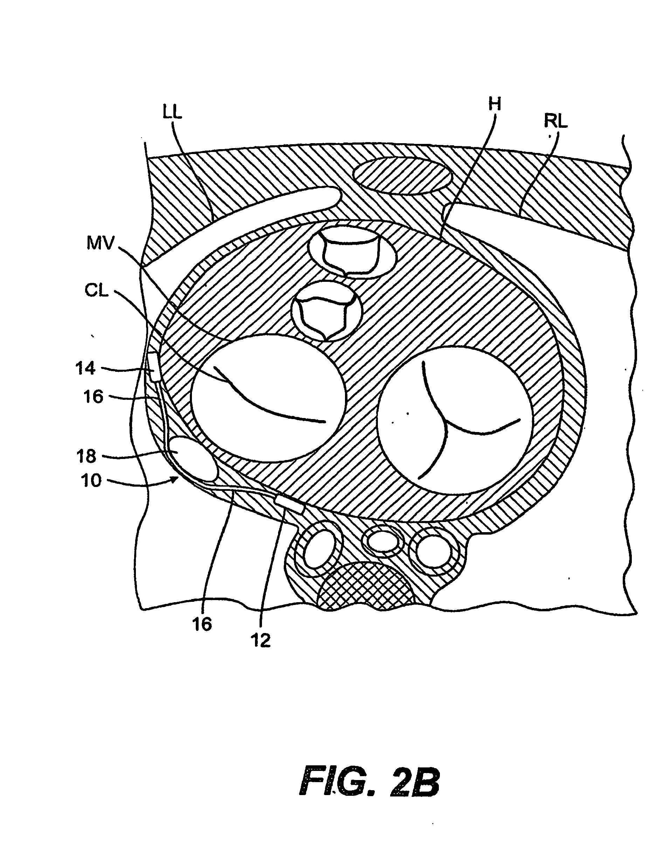

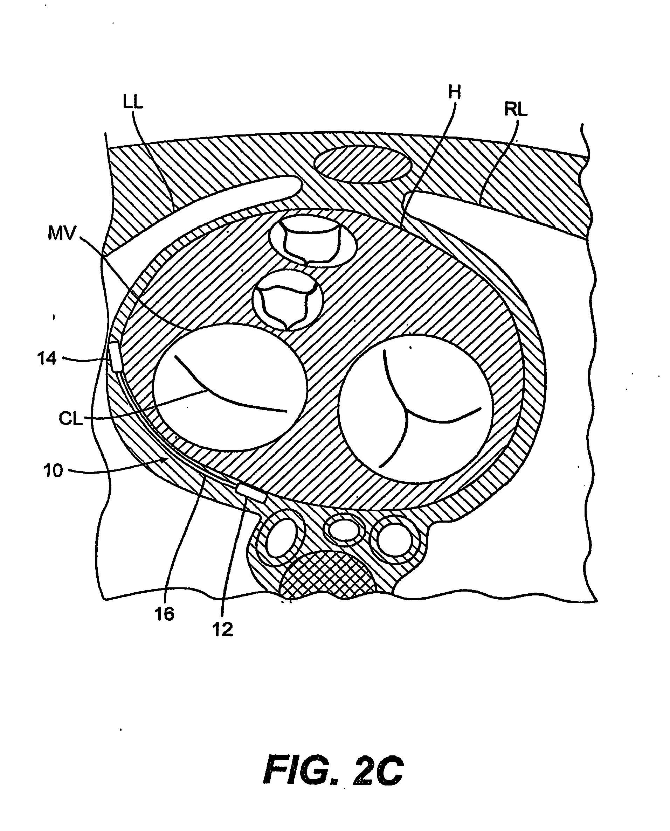

With reference to Figure 2A - 2C, cross sectional views of a patient's trunk at the level of the mitral valve MV of the heart H show the effects of implantable device 10 on mitral valve MV function. As seen in Figure 2A, an incompetent mitral valve MV is shown during systole, as rendered incompetent by, for example, a dilated valve annulus AN, a displaced papillary muscle PM due to ventricular dilation or other mechanism. With reference to Figures 2B and 2C, the implantable device 10 may be positioned outside and adjacent the heart wall such that the device 10 acts on the mitral valve MV. As seen in Figures 2B and 2C, the formerly incompetent mitral valve MV is shown during systole as corrected with implantable device 10. The implantable device 10 causes inward displacement of a specific portion of the heart wall adjacent the mitral valve MV resulting in reconfiguration and re-shaping of the annulus AN and/or the papillary muscles PM, thus providing more complete closure of the mitral valve leaflets AL/PL during systole, as shown by closed coaptation line CL in Figures 2B and 2C.

The implantable device 10 may affect MV function by acting on the adjacent heart wall in several different modes. For example, in one mode of operation, the protrusion 18 (or the interconnecting member 16 if no protrusion is used) of the implantable device 10 may apply or focus an inward force against the heart wall acting on the MV. The back-up force (i.e., the substantially equal and opposite force to the inward force) may be provided by the interconnecting member 16 as fixed to the heart wall by the anchor ends 12/14, the anatomical structure behind the protrusion 18, or a combination thereof. In an alternative mode of operation, the implantable device 10 may act to cinch, compress or otherwise deform the heart wall surrounding the posterior aspect of the mitral valve MV by shortening the circumferential length thereof. In another alternative mode of operation, the implantable device 10 acts to both apply an inward force and cause circumferential shortening. In each of these modes of operation, the inward force and/or circumferential shortening may be applied throughout the cardiac cycle, or may only act during a portion of the cardiac cycle such as during systole.

The implantable device 10 may be implanted in a number of different positions, a select few of which are described herein for purposes of illustration, not necessarily limitation. Generally, the implantable device 10 may be positioned, outside the epicardium of the heart wall adjacent the mitral valve MV, such as between the epicardium and pericardium, or between the pericardium and the pleural sac. Also generally, to maximize the effectiveness of the inward force, the implantable device 10 may be positioned to create a normal force against the heart wall that is generally orthogonal to the coaptation line CL formed by the leaflets PL/AL. This may be achieved, for example, by positioning the device 10 in a posterior-lateral projection of the mitral valve MV generally orthogonal to the middle tangent of the coaptation line CL as shown in Figures 2B and 2C.

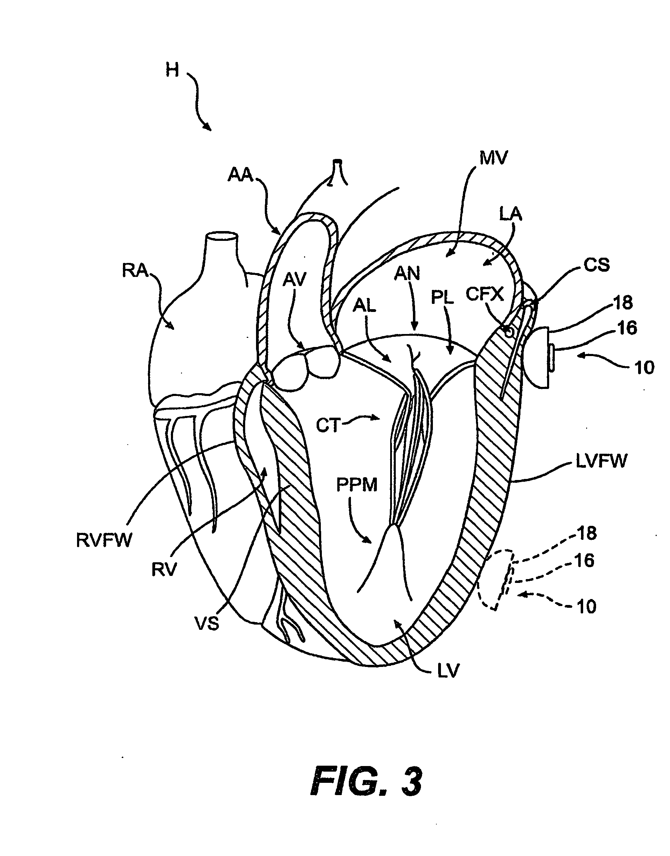

A variety of long axis and short axis positions are contemplated and the particular combination may be selected to have the desired effect. In the short axis view as seen in Figures 2B and 2C, the implantable device 10 may extend along all of, a portion of, or beyond the posterior-lateral projection of the mitral valve MV. In the long axis view as seen in Figure 3, the implantable device 10 may extend along all of, a portion of, or beyond the posterior-lateral projection of the mitral valve MV structures, including the papillary muscles PM, the chordae CT, the leaflets PL/AL, and the annulus AN. For example, the implantable device 10 may be positioned adjacent the annulus AN (e.g., extending slightly above and below the annulus AN near the AV groove), or adjacent the papillary muscles PM (e.g., extending slightly above and below the papillary muscles PM).

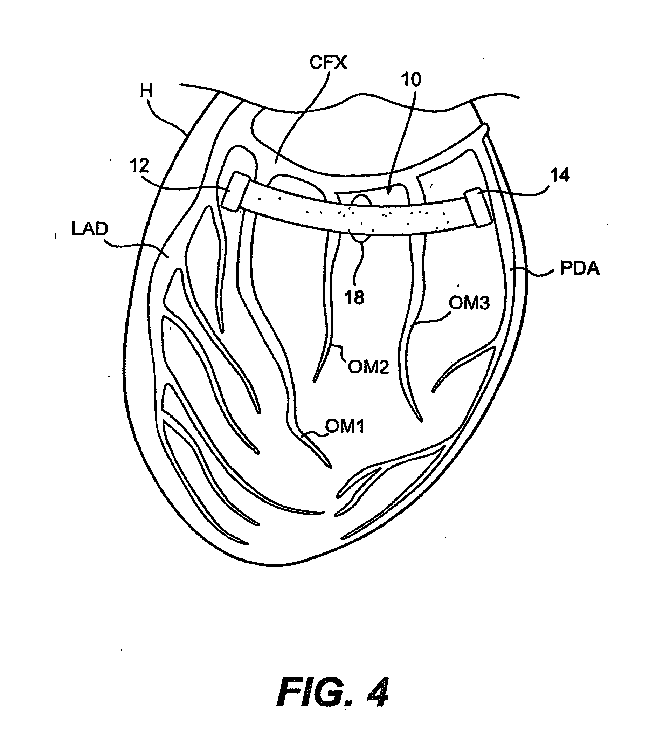

To avoid compression of the coronary arteries which typically reside near the surface of the heart wall, the implantable device 10 may have relatively small contact areas selected and positioned to establish contact with the heart wall while avoiding key anatomical structures. For example, as shown in Figure 4, the implantable device 10 may be positioned with the first anchor 12 positioned between the proximal left anterior descending artery LAD and the proximal first obtuse marginal OM1, the protrusion positioned inferior of the circumflex artery CFX between the second obtuse marginal OM2 and third obtuse marginal OM3, and the second anchor 14 positioned adjacent the posterior descending artery PDA. Alternatively, the implantable device 10 may have a relatively large surface area in contact with the heart wall to distribute the applied forces and avoid force focal points, particularly on the cardiac vasculature.

**Description of Exemplary Delivery Techniques and Approaches**

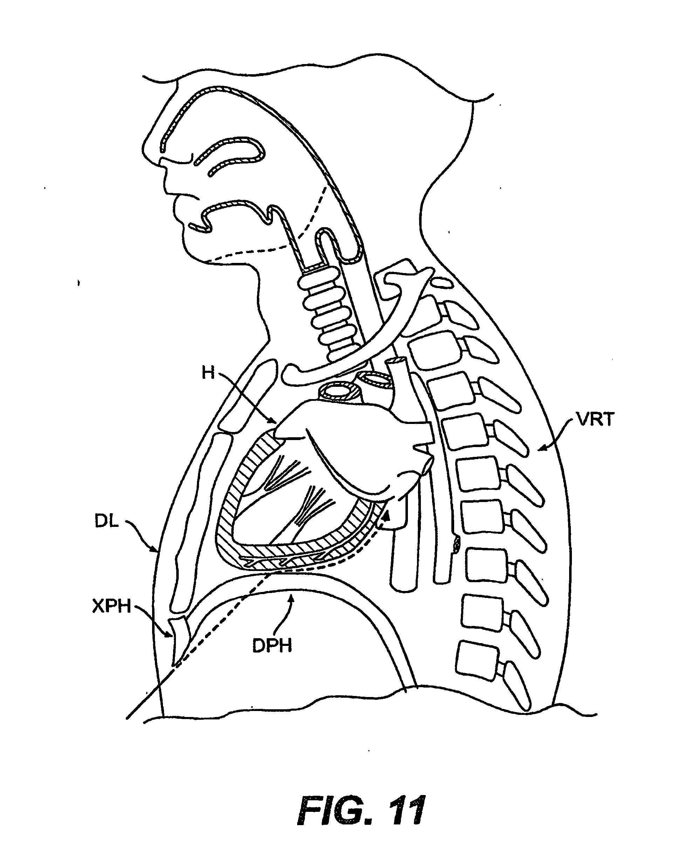

The implantable device 10 may be implanted using one or a combination of various methods and approaches. Generally, these delivery methods may be utilized to implant the device 10 in the pericardial space adjacent the posterior projection of the mitral valve MV. There are a number of different approaches and techniques for positioning the implantable device 10 as such, and these generally include surgical, transluminal and transthoracic techniques. For purposes of illustration, not necessarily limitation, an anterior transthoracic (subxiphoid) approach is described in more detail with reference to Figure 11. Examples of other suitable approaches are described in more detail by Vidlund et al., `519.

**Exemplary Embodiments of Implant Devices**

With reference to Figures 5A - 5D, perspective views of implantable devices 110, 210, 610 and 710, respectively, are shown. Note that the side of the device 110/210/610 that faces the heart wall when implanted is the top side in the illustration. Devices 110, 210, 610 and 710 are further exemplary embodiments of the generic embodiment of implantable device 10 described previously, in which similar components have similar nomenclature, and such may be made, used and function in the same or similar manner.

As seen in Figure 5A, implantable device 110 includes a first anchor 112, a second anchor 114, a interconnecting member 116, and an optional protrusion 118. Each of the first anchor 112, second anchor 114, interconnecting member 116, and protrusion 110 may be loaded with a radiopaque material to render the visible under x-ray. In this embodiment, the interconnecting member 116 may comprise cables 132 and 134, and the anchors 112 and 114 may comprise vacuum cups 120 with tissue piercing pins 122, as will be described in more detail hereinafter. The anchor members 112 and 114 may be selectively attached, released and re-attached to the heart, and the protrusion 118 may be selectively adjusted relative to the anchor members 112 and 114 by adjusting the respective lengths of the interconnecting member 116. The ends of the interconnecting member 116 may be fixedly attached to the anchors 112 and 114, and adjustment of the length of the interconnecting member 116 is provided by a locking mechanism 160 as seen in and described with reference to Figure 6A.

The anchors 112 and 114 may comprise a vacuum cup 120 with a tissue piercing pin 122 extending through the interior thereof. The cup 120 may be injection molded, for example, of a suitable biocompatible material such as PEEK, HDPE or PTFE, and the piercing pins 122 may be formed of stainless steel, for example. The piercing pins 122 are slidingly received in two bores disposed in the walls of the cup 120. A locking mechanism such as mating geometry between the bores and the pins may be used to lock the pins in the pierced position as shown. A port 124 in communication with the interior of the cup 120 is provided for releasable connection to an anchor catheter 400 as shown and described with reference to Figures 6A and 6B.

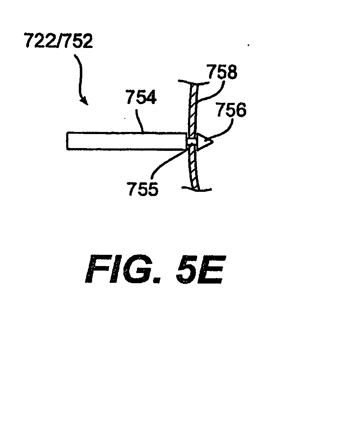

Each cup 120 has a rim that conforms to the epicardial surface of the heart wall such that vacuum applied to the cup 120 by the anchor catheter 400 via port 124 draws the epicardial surface of the heart into the interior of the cup. With the epicardial tissue drawn inside the cup by the vacuum, the tissue piercing pin 122 may be advanced to pierce through the heart tissue and lock in the pierced position as shown. A lock mechanism such as illustrated in Figure 5E may be used to secure pins 122. In this manner, the anchors 112 and 114 may be secured to the outside surface of the heart wall.

The protrusion 118 includes a base 140, an inflatable balloon 142 mounted to the base 140, and an outer covering 144 (shown partially cut-away) extending over the balloon 142. The base 140 may be connected to a locking mechanism 160 (not visible) located on the opposite side of the balloon 142, which in turn is connected to the interconnecting member 116. The base 140 may comprise a flexible or semi rigid polymeric material, and the balloon 142 may comprise a compliant or non-complaint polymeric material conventionally used for implantable balloons. Outer covering 144 may comprise a material that promotes tissue in-growth to provide additional anchoring stability over time. The balloon 142 may be prefilled, or may be filled during implantation, with a liquid that may solidify (cured) over time. To facilitate inflation of the balloon 142, the interior of the balloon 142 may be in fluid communication with an inflation catheter via a lumen (not visible) extending through the locking mechanism 160 and the base 140 as described with reference to Figure 6A.

The interconnecting member 116 may comprise two multifilament braided cables 132 and 134. One end of each cable 132 and 134 may be fixedly connected to the anchors 112 and 114, respectively, and the other ends of the cables 132 and 134 may be adjustably connected together by a locking mechanism 160 (not visible) attached to the base 140 of the protrusion 118. The cables 132 and 134 may extend through a pair of atraumatic pads 130 that are secured to the base 140 of the protrusion 118.

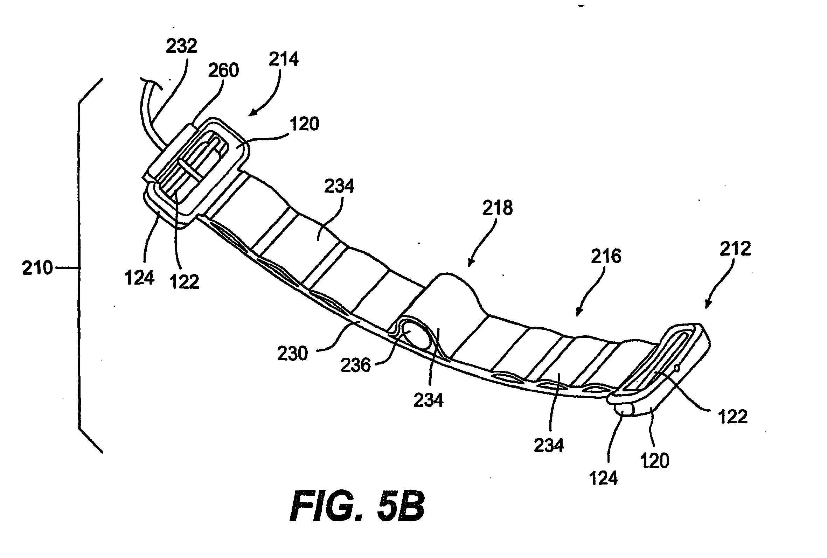

As seen in Figure 5B, implantable device 210 includes a first anchor 212, a second anchor 214, a interconnecting member 216, and a protrusion 218. In this embodiment, the interconnecting member 216 includes a cable 232 extending through a strap 230, with one end of the cable 232 fixedly connected to first anchor 212, and the other end extending through second anchor 214 to which the cable may be selectively locked to adjust the length of the interconnecting member 216. A locking mechanism 260, similar to the locking mechanism 160 discussed with reference to Figure 6A, may be connected to the second anchor 214 for selective tightening of and fixation to cable 232. Otherwise, anchors 212 and 214 may be the same as anchors 112 and 114 described previously.

Strap 230 may vary in length as a function of the length of the cable 232, and includes a plurality of pockets 234 that may be selectively filled with one or more plugs 236 to serve as the protrusion 218, or the pockets 234 may remain empty. For example, selection of the pockets 234 to fill with plugs 236 may be made apply an inward force against the heart wall while avoiding or jumping over coronary arteries residing near the surface of the heart wall. Strap 230 may comprise a woven polymeric material as polyester, and the plug 236 may comprise a solid polymeric material such as PEEK, silicone, HDPE, PTFE or ePTFE.

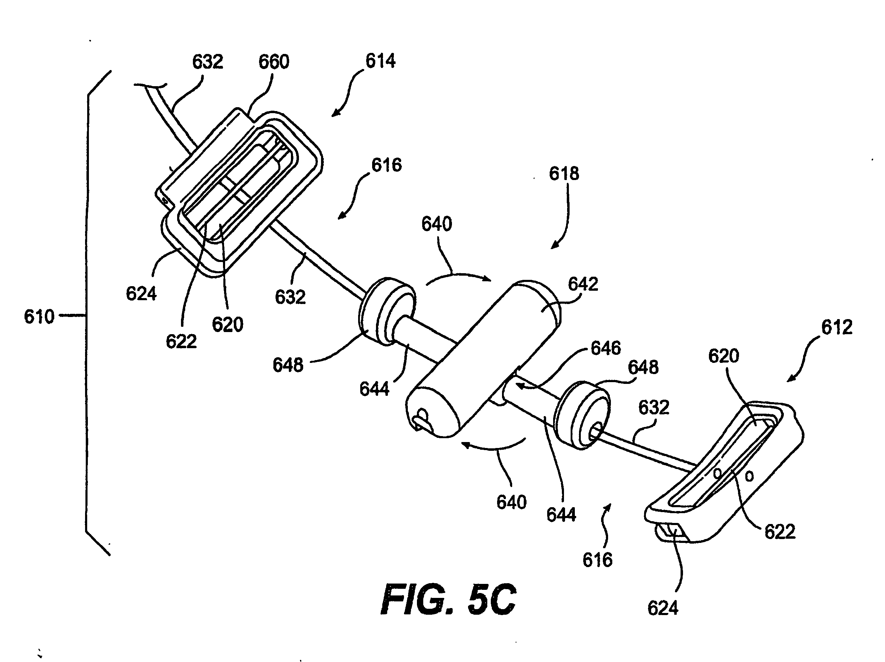

As seen in Figure 5C, implantable device 610 includes a first anchor 612, a second anchor 614, a interconnecting member 616, and a protrusion 618. In this embodiment, the interconnecting member 616 includes cable 632 extending through protrusion 618, with one end of the cable 632 fixedly connected to first anchor 612, and the other end extending through second anchor 614 to which the cable may be selectively locked to adjust the length of the interconnecting member 616. A locking mechanism 660, similar to the locking mechanism 160 discussed with reference to Figures 6A and 5E, may be connected to the second anchor 614 for selective tightening of and fixation to cable 632. Anchors 612 and 614 include interior cavities 620 in fluid communication with a vacuum source to accommodate heart tissue for securement thereto by tissue piercing pins 622. A port 624 in communication with the interior of the cup 620 is provided for releasable connection to an anchor catheter 400 or 800 as shown and described with reference to Figures 6A/6B and Figure 7, respectively. Recesses may be provided in each of the anchors 612 and 614 and the protrusion 618 for attachment of tissue in-growth promoting material such as Dacron fabric attached by suture-like material to cover the top, bottom and side surfaces. Otherwise, anchors 612 and 614 may be the same as anchors 112 and 114 described previously.

Protrusion 618 may include a center rotating member 642 coupled to cross member 644 by pivot connection 646. The rotating member 642 may be rotated 90 degrees relative to cross member 644 about pivot 646 as indicated by arrows 640. The rotating member 642 may be rotated as indicated by arrows 640 between a low profile delivery configuration wherein the rotating member 642 is generally aligned with the cross member 644, and a deployed configuration wherein the rotating member 642 is generally orthogonal to the cross member 644 as shown. The rotating member 642 may be rotationally biased to the deployed configuration and may be locked in the deployed configuration. A pair of protrusions 648 may be disposed at opposite ends of the cross member 644. The rotating member 642 in addition to the protrusions 648 may function as protrusions as described previously, while the gap therebetween may be used to avoid critical anatomical structures such as coronary vasculature.

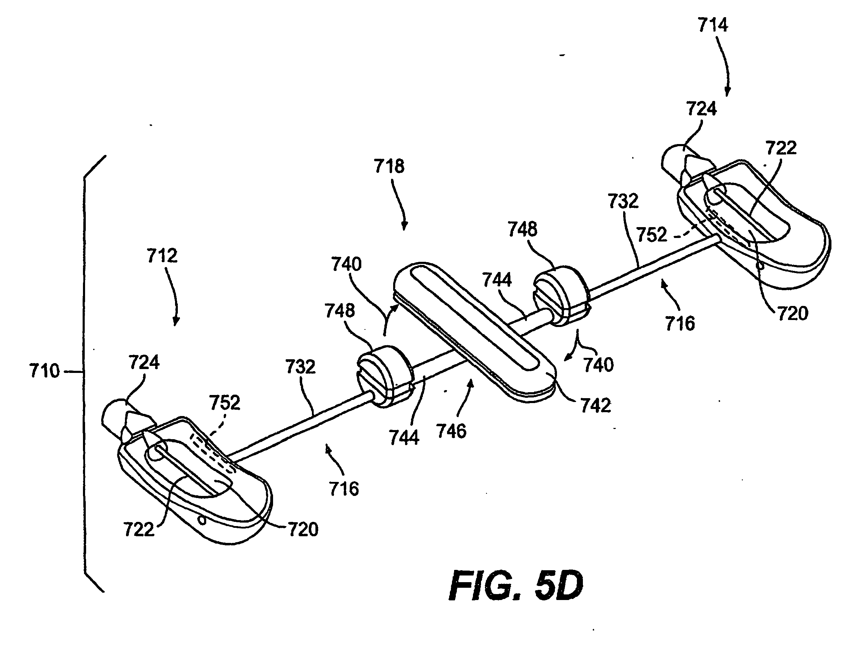

As seen in Figure 5D, implantable device 710 includes a first anchor 712, a second anchor 714, a interconnecting member 716, and a protrusion 718. In this embodiment, the interconnecting member 716 includes cable 732 fixedly attached to and extending through protrusion 718, with both ends of the cable 732 adjustably connected to the anchors 712 and 714 by pins 752 to selectively lock and adjust the length of the interconnecting member 716. Anchors 712 and 714 include interior cavities 720 in fluid communication with a vacuum source to accommodate heart tissue for securement thereto by tissue piercing pins 722. A port 724 in communication with the interior of the cup 720 is provided for releasable connection to an anchor catheter 400 or 800 as shown and described with reference to Figures 6A/6B or Figure 7, respectively. Recesses may be provided in each of the anchors 712 and 714 and the protrusion 718 for attachment of tissue in-growth promoting material such as Dacron fabric attached by suture-like material to cover the top, bottom (inside anchor) and side surfaces (away from heart surface). Otherwise, anchors 712 and 714 may be the substantially the same as anchors 112 and 114 described previously.

Protrusion 718 may include a center rotating member 742 coupled to cross member 744 by pivot connection 746. The rotating member 742 may be connected to the cross member 744 by an elastic ring and may be rotated 90 degrees relative to cross member 744 about pivot 746 as indicated by arrows 740. The rotating member 742 may be rotated as indicated by arrows 740 between a low profile delivery configuration wherein the rotating member 742 is generally aligned with the cross member 744, and a deployed configuration wherein the rotating member 742 is generally orthogonal to the cross member 744 as shown. The rotating member 742 may be rotationally biased to the deployed configuration and may be locked in the deployed configuration. A pair of protrusions 748 may be disposed at opposite ends of the cross member 744. The rotating member 742 in addition to the protrusions 748 may function as protrusions as described previously, while the gap therebetween may be used to avoid critical anatomical structures such as coronary vasculature.

As seen in Figure 5E, an example of a lock mechanism is shown to secure tissue piercing pins 722 and/or cable piercing pins 752. The pins 722/752 may include a cylindrical shaft 754 and a sharpened tip 756 with a recess 755 therebetween. A braided multifilament material 758 such as Spectra^{™} is provided distal of the pins 722/752 in the anchor housing 712/714 to catch the recess 755 of the pins 722/752 when the tip 756 is advanced therethrough. This effectively locks the pins 722/752 in the advanced position to secure the interconnecting member 716 to the anchors 712 and 714 and/or to secure the anchors 712 and 714 to the heart tissue as will be described in more detail hereinafter.

**Exemplary Embodiments of Delivery Devices**

With reference to Figure 6A, an example of a delivery system for delivery and implanting device 110 is shown. The delivery system generally includes a delivery catheter 300 and two anchor catheters 400, all of which are releasably connected to the implantable device 110. The illustrated delivery system is particularly suitable for delivering implantable device 110, but may also be modified for delivery of implantable devices 210, 610 and 710. The delivery system may be configured in terms of size, length, flexibility, radiopacity, etc., to facilitate a transthoracic delivery approach such as the subxiphoid delivery approach described with reference to Figure 11.

The delivery catheter 300 includes a tubular shaft 310 defining an inflation lumen and two cable lumens extending therethrough. A pair of push tubes 312 extend along side the tubular shaft 310 and slidably accommodate push rods 332 and 334. The distal ends of the tubular shaft 310 and push tubes 312 are coupled to the locking mechanism 160 by a release mechanism 326 such as a threaded, pinned or other releasable connection, such as the pin mechanism illustrated in Figure 5E. The push rods 332 and 334 may be advanced or retracted to selectively actuate individual pins 162 and 164 respectively in the lock mechanism 160 such that the pins 162 and 164 pass through the cables 132 and 134, respectively, and thus lock the cables relative thereto. Reference may be made to published U.S. Patent Application No. 2003/0050529 to Vidlund et al., the disclosure of which is incorporated herein by reference, for an example of a similar locking mechanism.

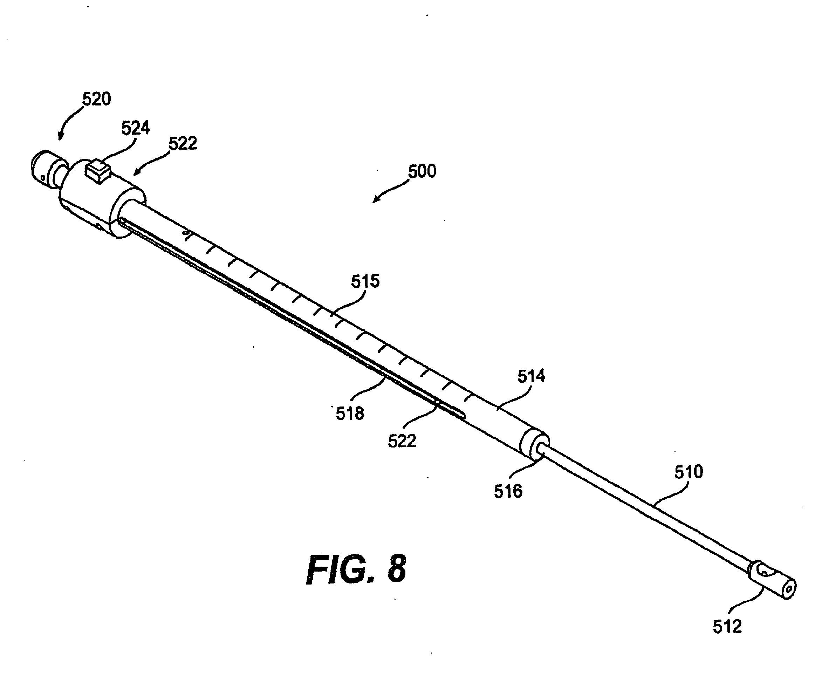

The proximal end of the tubular shaft 310 is connected to a manifold including connectors 322 and 324 and inflation port 318. The inflation lumen of the tubular shaft 310 provides fluid communication between the interior of the balloon 142 and the inflation port 318 of the manifold 314 for connection to an inflation device (not shown) to facilitate inflation and deflation of the balloon 142. If no balloon 142 is used, the inflation lumen and associated parts may be eliminated. The cable lumens of the tubular shaft 310 accommodate the proximal portions of the cables 132 and 134 for connection to a sizing device 500 via connectors 322 and 324 as described with reference to Figure 8, and for positioning the implant 110 relative to the anchors 112 and 114.

With additional reference to Figure 6B, the anchor catheters 400 are essentially mirror constructions of each other, and include a tubular shaft 410. A slit guide tube 412 extends along side a portion of the tubular shaft 410 to guide the cable 132/134 before the delivery catheter 300 is advanced as will be discussed in more detail hereinafter. A proximal end of the tubular shaft 410 is connected to a manifold 418 including a vacuum port 416 and a gasketed port 415 containing a push rod 414. A distal end of the tubular shaft 410 is releasably connected to the anchor 112/114 by a release mechanism 420 that may comprise a threaded, pinned or other releasable connection, for example. The tubular shaft 410 includes a vacuum lumen (not visible) extending therethrough to provide a fluid path from the interior of the cup 120 to the vacuum port 416 to facilitate connection to a vacuum source. The push rod 414 is disposed in the vacuum lumen of the catheter shaft 410 and may be slid therethrough to selectively advance or retract the piercing pin 122 in the cup 120.

With reference to Figure 7, an example of a delivery system for delivery and implanting device 710 is shown. The delivery system generally includes a two anchor catheters 800, both of which are releasably connected to the implantable device 710. The illustrated delivery system is particularly suitable for delivering implantable devices 210, 610 and 710, but may also be modified for delivery of implantable device 110. The delivery system may be configured in terms of size, length, flexibility, radiopacity, etc., to facilitate a transthoracic delivery approach such as the subxiphoid delivery approach described with reference to Figure 11.

The anchor catheters 800 are essentially mirror constructions of each other (with the exception of split tube 813), and include a tubular shaft 810 comprising a directional catheter construction connected to a handle 850. The directional catheter shaft 810 and associated handle 850 are available from Medamicus, Inc. of Plymouth, Minnesota. Handle 850 generally includes a grip portion 852 and a thumb knob 854 which actuates control wires in the directional catheter shaft 810 to permit selective bi-directional lateral deflection of the distal end thereof. The directional catheter shaft 810 and associated handle 850 accommodate a push rod (not visible) extending therethrough for actuation of the tissue piercing pin 722. The push rod for the tissue piercing pin 722 may comprise a stainless steel mandrel, for example, with a distal end abutting the proximal end of the tissue piercing pin 722, and a proximal end connected to a knob 814. The directional catheter shaft 810 and associated handle 850 also accommodate a vacuum lumen (not visible) extending therethrough to define a fluid path to the interior 720 of the anchor 712/714, such that a vacuum source (not shown) may be connected to vacuum port 816 on the handle 850 to provide suction at the anchor 712/714 to facilitate stabilization and securement to the outside of the heart wall.

Each of the anchor catheters 800 also includes a side tube 812 coextending with the directional catheter shaft 810. Side tube 812 accommodates the interconnecting member 732, a push rod (not visible) for actuation of the interconnecting member piercing pin 752, and a pull wire (not visible) for release of the anchor 712/714 as described in more detail below. The interconnecting member 732 extends through the side tube 812 from a proximal port 822/824 through the anchor 712/714 to the protrusion 718. To accommodate the interconnecting member 732 during initial delivery of the implant 710, a slotted side tube 813 may be provided on one of the catheters 800.

The push rod for the interconnecting member piercing pin 752 may comprise a stainless steel mandrel, for example, with a distal end abutting the proximal end of the interconnecting member piercing pin 752, and a proximal end connected to knob 832/834. A pair of guide loops 815 may be provided distal of the side tube to guide the interconnecting member 732, and a guide tube 862/864 may be provided distal of the side tube 812 to guide the push rod for the interconnecting member piercing pin 752.

The distal end of the directional catheter shaft 810 is connected to anchor 712/714 by a releasable connection 820, which may comprise a threaded type connection or a cotter pin type connection, for example. In the illustrated embodiment, the releasable connection 820 comprises a cotter pin type connection, with the pull wire (not visible) proximally connected to pull knob 842/844, and distally extending through aligned holes (not visible) in the anchor 712/714 and in the fitting on the distal end of the directional catheter shaft 810. By pulling proximally on pull knob 842/844, the anchor 712/714 may be released from the distal end of the directional catheter shaft 810.

With reference to Figure 8, a sizing device 500 is shown for adjusting the tension of interconnecting member 116, 216, 616, or 716 and in particular cable members 132/134, 232, 632 or 732. Sizing device 500 includes an elongate interconnecting member receiving tube 510 having a distal end including an engagement member 512 and a proximal end 516 connected to a preferably clear measuring tube 514 having a measuring scale 515 marked thereon. An inner tube 518 is disposed in the measuring tube 514 and is connected to a proximal end of the cable member to be tensioned. A lock mechanism 522 and release button 524 (biased in locked position) are connected to the proximal end of the measuring tube 514 to selectively lock the inner tube 518 relative to the measuring tube. A pin 522 protruding from inner tube 518 extends through a slot in measuring tube 514 to prevent relative rotation. An indicator (not visible) on the inner tube 518 adjacent the pin 522 is visible through transparent measuring tube 514 to facilitate linear measurement relative to scale 515.

To connect the cable to the inner rod or tube 518, the cable 132/134, 232, 632 or 732 is threaded through receiving tube 510, through measuring tube 514, through the inner tube 518, and placed in a retaining mechanism 520 disposed on the inner tube 518. Engagement member 512 may be connected to one of the connectors 322/324 or 822/824 on the delivery catheter, or directly to the lock mechanism 160 of device 110 or lock mechanism of device 210. With this arrangement, the inner tube 518 may be pulled proximally relative to the measuring tube 514 to apply tension to the cable and thus selectively adjust the tightness or degree of cinching of the implantable device 110/210/610/710, and/or selectively adjust the position of the protrusion relative to the anchor ends.

**Exemplary Embodiments of Access Devices**

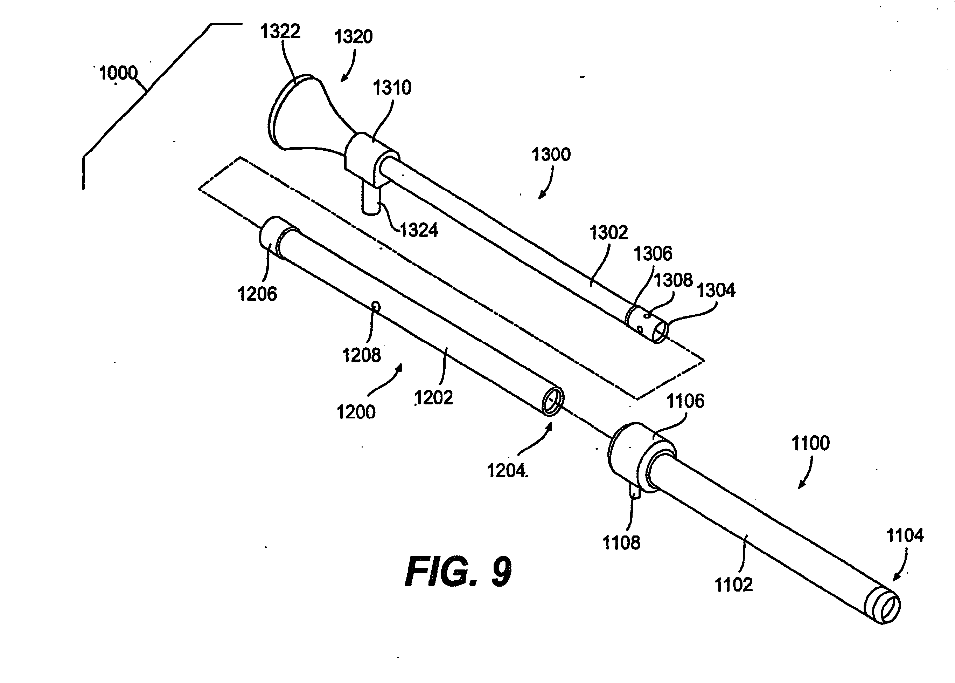

With reference to Figure 9, an exemplary embodiment of an access device 1000 is shown. Access device 1000 provides for less invasive surgical access from a point outside the patient's body, through a transthoracic port to the pericardial space around the patient's heart, as will be described in more detail with reference to Figure 11. A variety of pericardial access devices may be used to delivery the implantable device 10, and thus access device 1000 is shown by way of example not limitation. In this exemplary embodiment, access device 1000 includes an outer tube 1100, a securement tube 1200, and a cutter tube 1300. The securement tube 1200 is slidably and coaxially disposed in outer tube 1100, and similarly, the cutter tube 1300 is slidably and coaxially disposed in the securement tube 1200.

Outer tube 1100 may comprise a rigid tubular shaft 1102 formed of stainless steel, for example, having a lumen extending therethrough. A cap 110.4 having an interior recess (not visible) may be connected to the distal end of the shaft 1102. A handle 1106 may be connected to a proximal end of the tubular shaft 1102 to facilitate manual manipulation. A vacuum port 1108 may be incorporated into the handle 1106 to facilitate connection to a vacuum source (not shown) for establishing a vacuum in the lumen extending through the tubular shaft 1102.

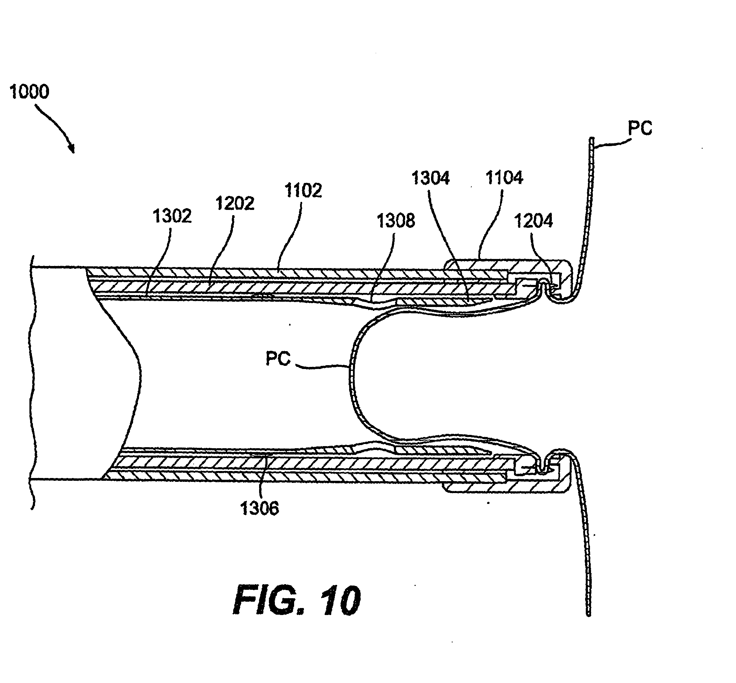

The securement tube 1200 may comprise a rigid tubular shaft 1202 formed of stainless steel, for example, having a lumen extending therethrough. An annular array of pericardium piercing pins 1204 may be disposed at the distal end of the tubular shaft 1202, and are sized to fit in the recess inside cap 1104 at the distal end of the outer tube 1100 as will be discussed in more detail with reference to Figure 10. A handle 1206 may be disposed at the proximal end of the tubular shaft 1202 to facilitate manual manipulation and to act as a stop to prevent the securement tube 1200 from advancing fully into outer tube 1100. A vacuum hole 1208 may be provided through the side of the tubular shaft 1202 to provide a fluid path from the interior of the outer tube 1100 to the interior of the securement tube 1200, thus permitting a vacuum to be established inside the tubular shaft 1202 of the securement tube 1200 by application of a vacuum to vacuum port 1108.

The cutter tube 1300 may comprise a rigid tubular shaft 1302 formed of stainless steel, for example, having a lumen extending therethrough. An annular cutting edge 1304 may be disposed at the distal end of the tubular shaft 1302. An annular ring 1306 may be disposed adjacent the distal end of the tubular shaft 1302 to provide a slidable fluid seal with the inside surface of the tubular shaft 1202 of the securement tube 1200. A series of vacuum holes 1308 may be provided through the side of the tubular shaft 1302 distal of the annular ring 1306 to provide a fluid path from the interior of the securement tube 1200 to the interior of the cutter tube 1300, thus permitting a vacuum to be established inside the tubular shaft 1302 of the cutter tube 1300 by application of a vacuum to vacuum port 1108. A handle 1310 may be disposed at the proximal end of the tubular shaft 1302 to facilitate manual manipulation and to act as a stop to prevent the cutter tube 1300 from advancing fully into securement tube 1200. A visualization device 1320 such as a camera or eye piece 1322 and light source 1324 may be connected to the proximal end of the tubular shaft 1302 to permit direct visualization down the lumen of the cutter tube 1300. Alternatively, an intracardiac echo device may be inserted therethrough, using vacuum for stability, to permit visualization and guidance on the epicardial surface.

With reference to Figures 9 and 10, the operation of the distal portion of the access device 1000 may be appreciated. The cutter tube 1300 and the securement tube 1200 may be disposed in the outer tube 1100 with the distal ends thereof slightly retracted. The outer tube 1100 may be inserted through a transthoracic port until the distal cap 1104 engages the pericardium (PC) surrounding the heart. Vacuum is applied to port 1108 thus drawing the PC into the lumen of the outer tube 1100, the securement tube 1200, and the cutter tube 1300 to form inward protrusion. The vacuum also draws the PC into the interior recess of the cap 1104 to form an annular fold. The securement tube 1200 may then be advanced distally until the array of pins 1204 passes through the annular fold in the PC, thus mechanically securing and sealing the PC to the access device 1000. The cutter tube 1300 may then be advanced distally until the annular cutting edge 1304 cuts the inward protrusion of, the PC, leaving the annular fold of the PC secured to the access device 1000. With the annular fold of the PC mechanically and sealingly connected to the distal end of the access device 1000, and with the outside diameter of the access device 1000 sized to form a seal in the transthoracic port, a sealed access path is established to the pericardial space that is isolated from the pleural space.

Exemplary Embodiments of Access and Delivery Methods

In Figure 11, a transthoracic anterior approach is shown as a dashed line with a distal arrow. This anterior approach may comprise a subxiphoid approach to establish access to the pericardial space, similar to the technique described by Kaplan et al. in U.S. Patent No. 6,423,051. An alternative lateral or posterior approach may utilize similar tools and techniques to access the pericardial space from the side or back between the ribs (intercostal space), similar to the techniques described by Johnson in U.S. Patent No. 5,306,234.

Generally speaking, once pericardial access is established with an access system as described with reference to Figures 9 and 10, a delivery system as described with reference to Figures 6 and 7 may be used to advance and manipulate the device 10 to the desired deployment position in the pericardial space adjacent the mitral valve MV or a specific part thereof. Assessment of the position and function of the device 10 relative to internal mitral valve MV structures such as leaflets AL/PL, papillary muscles PM, and regurgitant jet may be performed with ultrasonic imaging such as trans-esophageal, intracardiac or epicardial echocardiography, or x-ray fluoroscopy. These techniques may also be used monitor the adjustment of the size and/or tension of the device 10 with an adjustment device as described with reference to Figure 8 until the desired acute effect is established. Once in the desired position, the device 10 may be detached or otherwise disengaged from the distal end of the delivery system, which is subsequently removed.

The following detailed example of a delivery method using the delivery system and implant illustrated in Figure 7 is described by way of example, and may be applied to other delivery systems and implants described herein. This method may be broken down into six general steps: (1) establish pericardial access; (2) deliver the first anchor (e.g., near the PDA); (3) deliver the protrusion; (4) deliver the second anchor (e.g., near the LAD); (5) adjust the implant to achieve the desired effect on MV function; and (6) remove the delivery system leaving the implant in place on the outside of the heart.

To establish pericardial access, a needle may be inserted into the chest cavity below the xiphoid as generally shown in Figure 11. A guide wire (e.g., 0.035" diameter) may then be inserted into the needle and advanced toward the cardiac space. The needle may then be removed leaving the guide wire in place, and one or more dilators may then be advanced over the guide wire to dilate the percutaneous path: The dilator(s) may then be removed, and the access device illustrated in Figure 9 may be advanced over the wire adjacent the pericardium. Fluoroscopic visualization (e.g., AP and lateral views) may be used to confirm the desired pericardial access site.

Using the access device illustrated in Figure 9, vacuum may be applied to cause the pericardium to be sucked into the distal end thereof, and the tissue piercing pins may be actuated to mechanically secure the pericardium to the access device. The cutter tube may then be advanced to cut and remove a portion of the pericardium in the distal end of the access device, thus establishing a path from the exterior of the body to the pericardial space around the heart.

Initially, the interconnecting member may be loaded into the first anchor and anchor catheter with one side of the interconnecting member extending through the side tube and the other side of the interconnecting member extending through the slotted side tube. Before delivering the anchor, angiographic visualization of the left and/or right coronary arteries may be performed to map the locations of the critical arteries. To deliver the first anchor near the PDA as shown in Figure 4, for example, the anchor catheter may be manipulated through the access device until the anchor is adjacent the PDA near the last obtuse marginal (OM3), using fluoroscopic visualization to aid navigation. After ascertaining that the first anchor is not positioned over any coronary arteries, vacuum may be applied to the first anchor to temporarily stabilize the anchor on the outside of the heart wall and to pull tissue into the interior of the anchor. The tissue piercing pins may then be actuated to secure the first anchor to the heart wall.

The protrusion may then be advanced along the first anchor catheter by removing one end of the interconnecting member from the slotted tube on the anchor catheter, inserting it through the protrusion and fixing the protrusion midway on the interconnecting member. A delivery tube may be placed about the protrusion to retain it in the delivery configuration, and the delivery tube with the protrusion therein may then be inserted through the access device. By pulling on the opposite end of the interconnecting member and by manipulating the delivery tube, the protrusion may be advanced until it is adjacent the first anchor.

Before delivering the second anchor near the LAD as shown in Figure 4, the interconnecting member may be inserted into the second anchor and through the side tube of the second anchor catheter. The second anchor may then be slid over the interconnecting member using the anchor catheter, passing through the access device and into the pericardial space. With the aid of fluoroscopic guidance, the second anchor may be positioned next to the junction of the LAD and CFX as seen in Figure 4, for example. After ascertaining that the second anchor is not positioned over any coronary arteries, vacuum may be applied to the second anchor to temporarily stabilize the anchor on the outside of the heart wall and to pull tissue into the interior of the anchor. The tissue piercing pins may then be actuated to secure the second anchor to the heart wall.

With the first and second anchors secured to the outside of the heart wall, and the protrusion extending therebetween, the interconnecting member may be tightened or cinched using the device illustrated in Figure 8, for example. MV function may be simultaneously observed using TEE or ICE, and the degree of cinching of the interconnecting member and/or the position of the protrusion may be adjusted to obtain the desired reduction in MV regurgitation (MVR).

With the aid of fluoroscopy, correct anchor positioning may be verified and adequate blood flow may be confirmed in the left coronary arteries. After confirming correct positioning and adequate reduction in MVR, the interconnecting members may be secured by actuating interconnecting member piercing pins with the associated push rods, and the directional catheter shaft may be disconnected from the anchors by actuating the releasable connection with the associated pull wires.

The delivery system may then be, removed, and the interconnecting members may be trimmed adjacent the anchors with a cutting device such as an elongate cautery tool. The access device may be removed and the sub-xiphoid access site may be closed using sutures.

**Alternative Delivery Approaches**

Various alternative approaches for delivering the implantable devices may be utilized. By way of example, the implant may be positioned outside the wall of the heart H adjacent the mitral valve MV to improve valvular function as described previously. The implant may be positioned outside the epicardium, such as between the epicardium and pericardium, or between the pericardium and the pleural sac, for example. There are a number of different approaches and techniques for positioning the implant as such, and these approaches generally include surgical, transluminal and transthoracic techniques. An example of a suitable surgical technique is conventional open heart surgery similar to that which is performed for coronary artery bypass surgery (CABG) or valve repair, which may be performed on-pump or off-pump. Examples of transluminal and transthoracic approaches are described below.

An example of a transluminal approach is via the coronary sinus CS. The coronary sinus CS may be catheterized by, for example, using a guide catheter and guide wire navigated through the inferior vena cava IVC or superior vena cava SVC from a convenient venous, access site such as a femoral, brachial or jugular approach. A guide catheter may be navigated into the right atrium RA and the distal end of the guide catheter may be seated in the ostium of the coronary sinus CS. A delivery catheter may be navigated through the guide catheter into the coronary sinus CS, with its distal end positioned near the desired exit point into the pericardial space. A guide wire may be advanced through the delivery catheter, out the distal end of the delivery catheter, and penetrate through the wall of the coronary sinus CS at the exit point. The delivery catheter may be advanced over the guide wire and through the hole in the coronary sinus CS and manipulated to the desired deployment position in the pericardial space adjacent the mitral valve MV or a specific part thereof.

The implant, which may be predisposed at the distal end of the delivery catheter or advanced to the distal end thereof, is then manipulated into the desired position and actuated, if necessary. The position of the implant may be monitored and confirmed using medical imaging techniques such as radiographic techniques, for example, with radiopaque material incorporated into the implant and/or the distal end of the delivery catheter. Upon deployment and actuation of the implant, assessment of the position of the implant relative to internal mitral valve MV structures such as leaflets AL/PL, papillary muscles PM, and regurgitant jet may be performed with ultrasonic imaging such as trans-esophageal, epicardial or intracardiac echocardiography. These techniques may also be used to refine the position of the implantable device until the desired acute effect is established. Once in the desired position, the implantable device may be detached or otherwise disengaged from the distal end of the delivery catheter, and the delivery catheter, guide wire and guide catheter may be removed. If desired, a catheter or small tube may remain permanently or temporarily attached to the implantable device to selectively adjust the tension of the interconnecting member or the size of the protrusion (e.g., by adding or removing material therefrom).

Another example of a transluminal approach is via a cardiac vein CV. This approach is similar to the coronary sinus CS approach described above except that the delivery catheter is navigated further through the coronary sinus CS and into a desirable cardiac vein CV near the desired implant site. The cardiac vein CV may be catheterized by, for example, using a guide catheter and guide wire navigated through the inferior vena cava IVC or superior vena cava SVC from a convenient venous access site such as a femoral, brachial or jugular approach. The guide catheter may be navigated into the right atrium RA and the distal end of the guide catheter may be seated in the ostium of the coronary sinus CS. The delivery catheter may be navigated through the guide catheter into the coronary sinus CS, into a cardiac vein CV, with its distal end positioned near the desired exit point into the pericardial space. The guide wire may be advanced through the delivery catheter, out the distal end of the delivery catheter, and penetrate through the wall of the cardiac vein CV at the exit point. The delivery catheter may be advanced over the guide wire and through the hole in the cardiac vein CV and manipulated to the desired deployment position in the pericardial space adjacent the mitral valve MV or a specific part thereof. Having established access to the desired implant position, the remaining steps for implantation may be the same or similar to those described above.

Yet another example of a transluminal approach is via the azygos vein AZV. The azygos vein AZV extends past the posterior aspect of the heart H near the left-right midline. The azygos vein AZV may be catheterized by, for example, using a guide catheter and guide wire navigated through the venous system from a convenient venous access site such as a femoral vein. The guide catheter may be navigated up to and adjacent the desired exit point adjacent the mitral valve MV or a specific part thereof. The delivery catheter may be navigated through the guide catheter until the distal end of the delivery catheter exits the distal end of the guide catheter and is positioned near the desired exit point. The guide wire may be advanced through the delivery catheter, out the distal end of the delivery catheter, and penetrate through the wall of the azygos vein AZV at the exit point. The delivery catheter may be advanced over the guide wire and through the hole in the azygos vein AZV and manipulated to the desired deployment position in the pericardial space adjacent the mitral valve MV or a specific part thereof. Having established access to the desired implant position, the remaining steps for implantation may be the same or similar to those described above.

A further example of a transluminal approach is via the right atrium RA. The pericardial space may be accessed via the right atrium RA using a percutaneous transatrial technique wherein the right atrium or right atrial appendage is catheterized by, for example, using a guide catheter and guide wire navigated through the inferior vena cava IVC from a convenient venous access site such as a femoral approach. The guide catheter may be navigated into the right atrium or atrial appendage and the guide wire may be used to puncture through the atrial wall to gain access to the pericardial space. The delivery catheter may be advanced over the guide wire and through the hole in the atrial wall and manipulated to the desired deployment position in the pericardial space adjacent the mitral valve MV or a specific part thereof. Having established access to the desired implant position, the remaining steps for implantation may be the same or similar to those described above.

Yet a further example of a transluminal approach is via the left ventricle LV. The pericardial space may be accessed via the left ventricle LV using a percutaneous transventricular technique wherein the left ventricle LV is catheterized by, for example, using a guide catheter and guide wire navigated through the ascending aorta AA from a convenient arterial access site such as a femoral approach. The guide catheter may be navigated into the left ventricle LV and the guide wire may be used to puncture through the ventricular wall to gain access to the pericardial space. The delivery catheter may be advanced over the guide wire and through the hole in the ventricular wall and manipulated to the desired deployment position in the pericardial space adjacent the mitral valve MV or a specific part thereof. Having established access to the desired implant position, the remaining steps for implantation may be the same or similar to those described above.

An alternative LV approach is across the atrial septum. The pericardial space may be accessed via the left ventricle LV using a percutaneous transventricular technique wherein the left ventricle LV is catheterized by, for example, using a guide catheter and guide wire navigated through the inferior vena cava IVC from a convenient venous access site. The guide wire may be navigated into the right atrium RA, through the atrial septum, into the left atrium LA, through the mitral valve MV, into the left ventricle LV, and punctured through the left ventricular wall to gain access to the pericardial space. The delivery catheter may be advanced over the guide wire and through the hole in the ventricular wall and manipulated to the desired deployment position in the pericardial space adjacent the mitral valve MV or a specific part thereof. Having established access to the desired implant position, the remaining steps for implantation may be the same or similar to those described above.

Another transluminal approach is via the left atrium LA. The pericardial space may be accessed via the left atrium LA using a percutaneous transatrial technique wherein the left atrium LA is catheterized by, for example, using a guide catheter and guide wire navigated through the inferior vena cava IVC from a convenient venous access site. The guide wire may be navigated into the right atrium RA, through the atrial septum, into the left atrium LA, and punctured through the left atrial wall to gain access to the pericardial space. The delivery catheter may be advanced over the guide wire and through the hole in the atrial wall and manipulated to the desired deployment position in the pericardial space adjacent the mitral valve MV or a specific part thereof. Having established access to the desired implant position, the remaining steps for implantation may be the same or similar to those described above.

Yet another transluminal approach is via the esophagus ES. The esophagus ES extends past the heart H near the posterior aspect of the right atrium. Because the esophagus ES does not provide a sterile environment, as isolation catheter may be used to isolate a portion of the esophageal lumen and establish a sterile environment. The isolation catheter may be inserted through nasal passage, past the pharynx, and into the esophagus ES. Alternatively, the isolation catheter may be inserted into the esophagus ES via the mouth. The distal portion of the isolation catheter may be positioned adjacent the heart H at the level of the mitral valve MV as confirmed by a suitable visualization techniques such as ultrasonic imaging (e.g., trans-esophageal, trans-thoracic, epicardial or intracardiac echocardiography). Once in the desired position, the isolation catheter may be actuated and the confined space may be flushed with a suitable sterilizing wash. Having established an isolated sterile environment, a guide wire may be advanced through the isolation catheter exiting near the isolated space and puncturing the esophageal wall at the desired exit point. The delivery catheter may be advanced over the guide wire and through the hole in the esophageal wall and manipulated to the desired deployment position in the pericardial space adjacent the mitral valve MV or a specific part thereof. Having established access to the desired implant position, the remaining steps for implantation may be the same or similar to those described above.

Examples of transthoracic approaches include anterior and posterior approaches. The anterior approach may comprise a subxiphoid approach to establish access to the pericardial space similar to the techniques described previously. The posterior approach may utilize similar tools and techniques to access the pericardial space from the back between the ribs and extending into the thoracic cavity. Once pericardial access is established with, for example, a thoracic guide catheter used in such techniques, a delivery catheter may be advanced over or together with a guide wire and manipulated to the desired deployment position in the pericardial space adjacent the mitral valve MV or a specific part thereof. Having established access to the desired implant position, the remaining steps for implantation may be the same or similar to those described above.

**Alternative Implants & Delivery Tools**

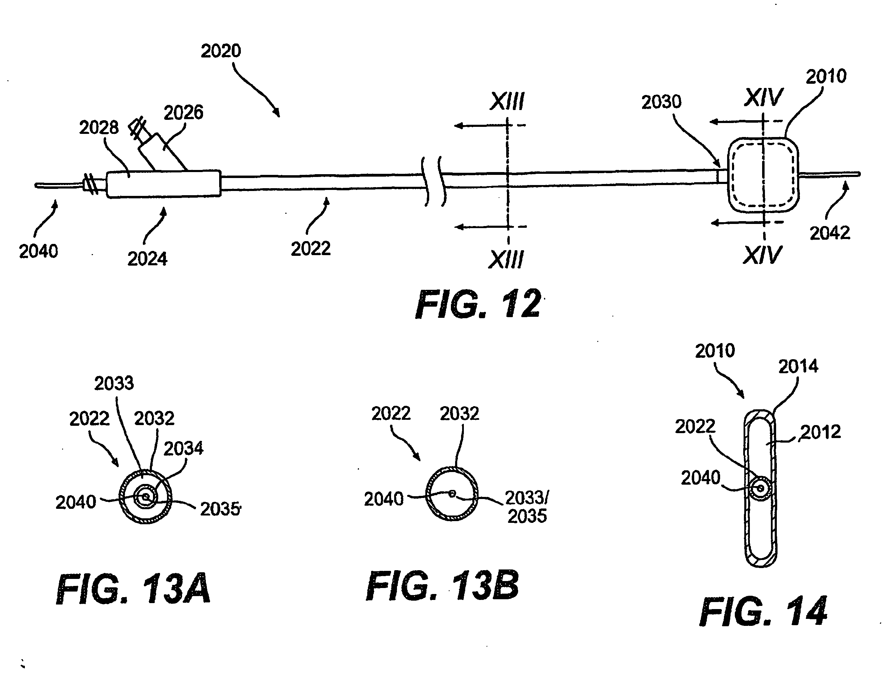

With reference to Figure 12, a schematic plan view of a delivery catheter 2020 and a guide wire 2040 is shown for use in delivering a implantable device 2010 by the transluminal techniques described above, for example. Delivery catheter 2020 includes an elongate shaft 2022 that is sized appropriately as a function of the delivery approach, both in terms of the size of the lumen and the distance from the access point to the deployment point. As seen in Figure 13A, the elongate shaft 2022 may comprise a coaxial over-the-wire design with an outer tube 2032 coaxially disposed about an inner tube 2034. The inner tube may define a guide wire lumen 2035 and the annular space between the outer tube 2032 and the inner tube 2034 may define an inflation lumen 2033. Alternatively, as seen in Figure 13B, the elongate shaft 2022 may comprise an innerless, semi-movable wire, or fixed-wire design with the outer tube 2032 coaxially disposed about the guide wire 2040, and a distal (movable, semi-movable or fixed) fluid seal provided between the distal end of the outer tube 2032 and a distal portion of the guide wire 2042. In this alternative design, the outer tube 2032 may define a combined guide wire lumen 2035 and inflation lumen 2033. In both designs, the outer tube 2032 includes an opening (not shown) to establish fluid communication with the interior 2012 of the implantable device 2010.

A manifold 2024 may be connected to the proximal end of the elongate shaft 2022 and may include an inflation lumen arm 2026 and a through lumen arm 2028. The inflation lumen arm 2026 is in fluid communication with the inflation lumen 2033 extending through the shaft 2022 and the interior 2012 of the implantable device 2010. The through lumen arm 2028 provides access for the guide wire 2040 to extend into the guide wire lumen 2035 through the shaft 2022 and through the implantable device 2010. The inflation lumen arm 2026 may be connected to an inflation device or other source of filler material such that material may be selectively added to or removed from the interior 2012 defined by wall 2014 of the implantable device 2010.

The implantable device 2010 may be releasably connected to a distal portion of the shaft 2022 by a release mechanism 2030 (shown schematically). The release mechanism 2030 may comprise a wide variety of forms known in the art related to detachable balloons and detachable coils. The release mechanism 2030 may be actuated at the proximal end of the catheter 2020 by an appropriate means depending on the type of release mechanism utilized. The release mechanism 2030 operates to secure the implantable device 2010 to the distal portion of the shaft 2022 during delivery until the implantable device 2010 is the desired deployment position. Once the implantable device is in the desired position and expanded, the release mechanism 2030 may be actuated to sever the connection between the delivery catheter 2020 and the implantable device 2010.

The guide wire 2040 may have sufficient length to extend through the delivery catheter, and sufficient flexibility and column strength to facilitate manipulation, navigation and tissue puncture capabilities. The size and shape of the distal tip 2042 of the guide wire 2040 may be selected as a function of what lumen need to be navigated and what tissue needs to be penetrated. For example, the distal tip 2042 may comprise a rounded tip having a diameter similar to a coronary guide wire to enable navigation through the vasculature and pericardial space, but with sufficient stiffness to puncture venous walls and atrial walls. Alternatively, the distal tip 2042 may have a smaller diameter or may be sharpened to puncture ventricular walls, esophageal walls, etc.

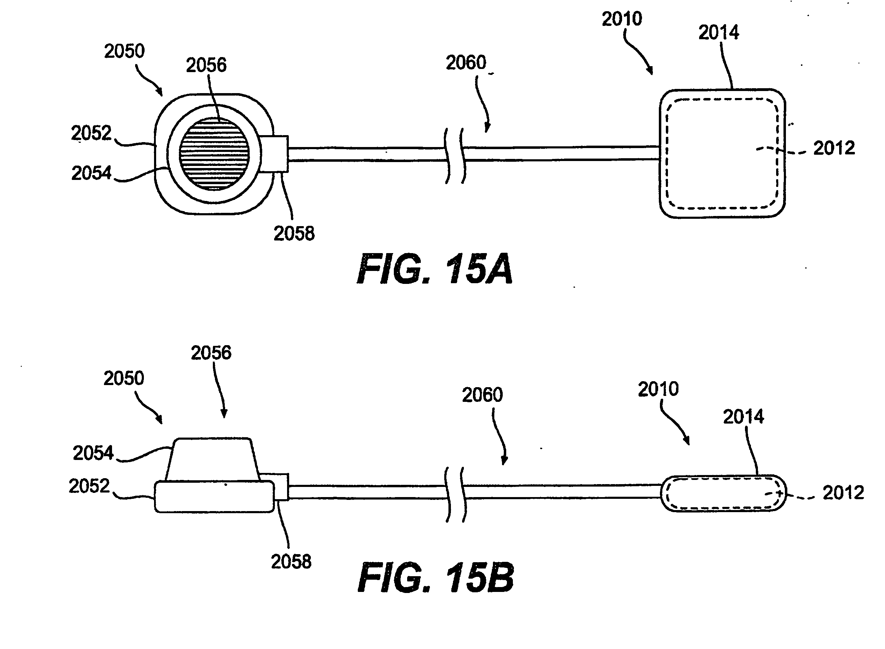

With reference to Figures 15A and 15B, schematic top and side views of a transdermal access port 2050 connected to a implantable device 2010 by a flexible catheter 2060. The transdermal access port 2050 may be used to selectively add or remove material to or from (e.g., inflate or deflate) the implantable device 2010 after the device 2010 has been deployed and the delivery procedure has been completed. For example, if the desired acute effect is achieved during deployment of the implantable device 2010, but thereafter the effect diminishes or otherwise changes in an undesirable way, it may be desirable to modify the size and/or shape of the implantable device 2010 by selectively adding or removing material form the device 2010 using the transdermal access port 2050.

The transdermal access port 2050 generally includes a base housing 2052 and a reservoir housing 2054 containing a reservoir (not visible) therein. A septum 2056 is disposed over the top of the reservoir in the housing 2054 and permits a needle to be inserted into the reservoir. The catheter 2060 is connected to the reservoir housing 2054 at strain relief 2058 and is in fluid communication with the reservoir therein. The transdermal access port 2050 may be implanted just below the dermal layer DL at a convenient access point such as in the pectoral region. The catheter 2060 extends from the subdermal location of the transdermal access port 2050 to the implantable device 2010 located adjacent the heart. With this arrangement, a needle may be used to inject fluid, for example, through the septum 2056 and into the reservoir of the transdermal access port 2050. From the reservoir of the transdermal access port 2050, the fluid passes through the flexible catheter 2060 and into the interior 2012 of the implantable device 2010 to increase its size and/or shape. In a similar manner, a needle may be used to withdraw fluid from the interior 2012 of the implantable device 2010 to decrease its size and/or shape. The catheter 2060 may be connected to the implantable device 2010 prior to deployment thereof and snaked to the transdermal access port 2050 via the delivery path defined by the delivery catheter or via an alternative route to the transdermal access port 2050, which may be surgically placed in a subdermal pocket. Alternatively, the catheter 2060 may be connected to the implantable device 2010 after deployment thereof.

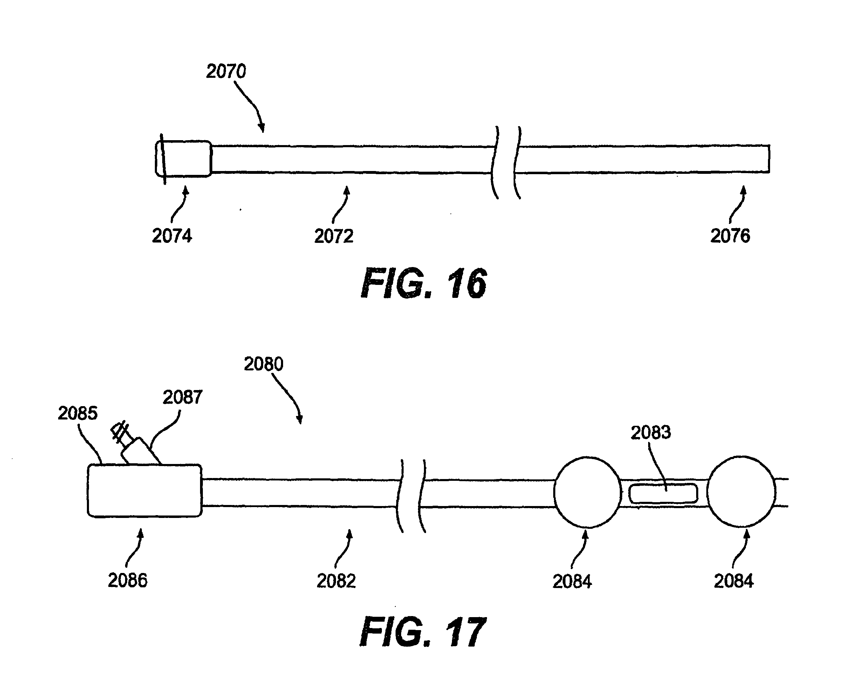

With reference to Figure 16, a schematic plan view of a guide catheter 2070 is shown, for use in delivering a implantable device 2010 by transluminal techniques, for example. The guide catheter 2070 includes an elongate shaft 2072 that is sized appropriately as a function of the delivery approach, both in terms of the size of the lumen and the distance from the access point to the deployment point. A hub 2074 may be connected to the proximal end of the shaft 2072 to facilitate insertion of a delivery catheter and/or guide wire, and to permit connection to a syringe for infusion of fluids such as radiopaque media. The construction of the shaft 2072 may be conventional, such as a multilayered design with composite braid and polymeric layers. The distal portion 2076 of the shaft 2072 may be curved with one or more curves in two or three dimensions to facilitate navigation and seating in the luminal path chosen. By way of example, not limitation, the guide catheter 2070 may comprise a commercially available 8 French multipurpose guide catheter.

With reference to Figure 17, a schematic plan view of an isolation catheter 2080 is shown, for use in delivering a implantable device 2010 by transluminal techniques, such as a transesophageal approach. The isolation catheter 2080 includes an elongate shaft 2082 that is sized appropriately as a function of the delivery approach, both in terms of the size of the lumen and the distance from the access point to the deployment point. For example, for a transesophageal approach, the shaft 2082 may have a diameter sized to fit into the esophagus ES and a length sufficient to extend from the nose or mouth to a point adjacent the heart H. The shaft 2082 includes a through lumen (not visible) for passage of a delivery catheter and/or guide wire, and a distal window or opening 2083 through which the space filling member 2010, the delivery catheter and/or the guide wire may exit the catheter 2080 between two balloons 2084, and through which a sterilizing wash solution may be flushed to aspirate the region between the balloons 2084. The shaft 2082 also includes an inflation lumen (not visible) to selectively inflate and deflate the balloons 2084. Upon inflation in the luminal passage (e.g., esophageal lumen), the balloons 2084 define an isolation zone therebetween that may be sterilized and isolated from the remainder of the passage. A manifold 2086 may be connected to the proximal end of the shaft 2082, and may include an inflation lumen arm 2087 in fluid communication with the inflation lumen in the shaft 2082 and a through lumen arm 2085 to provide access to the through lumen in the shaft 2082 and window 2083.

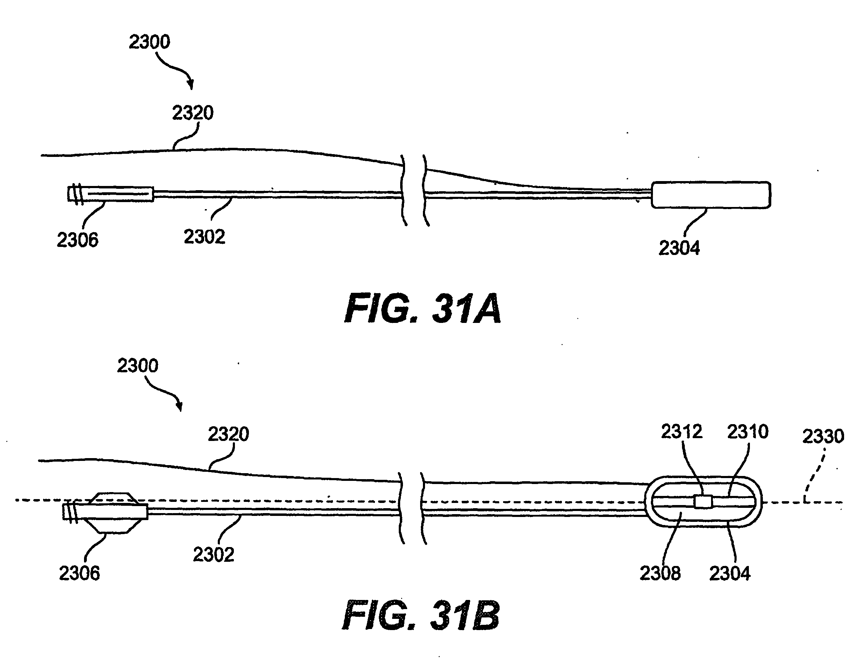

With reference to Figures 31A and 31B, side and top views, respectively, of anchor catheter 2300 are illustrated. Anchor catheter 2300 is particularly useful for delivering implantable devices by transthoracic techniques. Anchor catheter 2300 includes an elongate tubular shaft 2302 comprising a relatively rigid material such as stainless steel, NiTi, a braided composite. The elongate shaft 2302 may be straight or gently curved depending on the approach (subxiphoid or posterior). A suction cup 2304 may be connected to the distal end of the shaft 2302. The suction cup 2304 defines an interior 2308, and may have an open top and bottom, or an open bottom and closed top. For example, the suction cup 2304 may have an open top and bottom facing both the pericardium and epicardium, or an open bottom facing the epicardium and a closed top facing the pericardium.

The interior 2308 of the suction cup 2304 is in fluid communication with a vacuum lumen extending through the shaft 2302 to hub 2306, which may be connected to a vacuum source (not shown). A flexible guide wire 2320 extends alongside the shaft 2302, with its distal end connected to the suction cup 2304 and its proximal end free. A guide wire tube 2310 may extend through the suction cup 2304 to slidably accommodate pericardial space guide wire 2330 shown in phantom. A radiopaque marker 2312 may be disposed about the guide wire tube 2310 to facilitate visualization by radiography.

Pericardial space guide wire 2330 may be delivered into the pericardial space using a subxiphoid transthoracic cardiac access technique similar to that which is described by Schmidt et al. in U.S. Patent No. 6,206,004. The pericardial space guide wire 2330 provides access to the pericardial space, but typically has a free distal end and therefore may not be easily positioned or anchored in the desired location. Accordingly, the anchor catheter 2300 may be advanced over the pericardial space guide wire 2330, manipulated to the desired implant location using semi-rigid shaft 2302, and anchored in place using vacuum. Application of vacuum to suction cup 2304 effectively anchors the distal end of the catheter 2300 to the heart wall and permits delivery catheter 1900 (described hereinafter) to be advanced thereover.

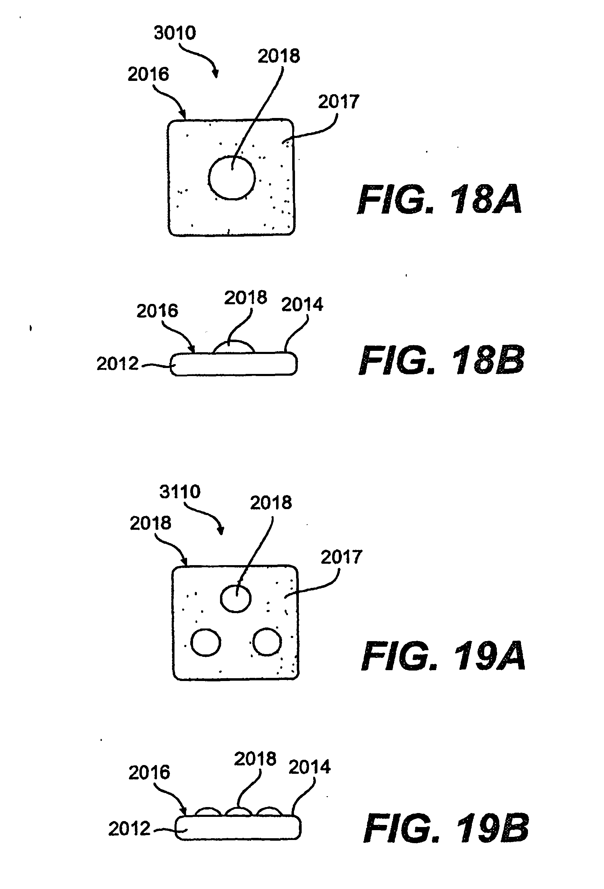

With reference to Figures 18 - 30, schematic illustrations of various design alternatives of implantable devices are shown. In Figures 18 - 23, a bottom view is shown in Figures labeled "A" and a side view (cross sectional in some) is shown in Figures labeled "B". The bottom view generally corresponds to the surface or surfaces facing the wall of the heart H and may lie directly against the epicardium, for example. The side view may represent a superior/inferior view, and/or a lateral view, depending on the selected orientation of the device. The size, shape and orientation of the implantable devices may be selected as a function of the implant site, such as the anatomical features associated with the implant site, and as a function of the desired effect(s) on valve function. The design alternatives schematically illustrated in Figures 18 - 25 are given by way of example, not limitation, and may be used individually or collectively.

Each implantable device described herein may have virtually any desired size, shape or configuration to meet the particular clinical requirements and to have the desired clinical effect(s) as described previously, some of which have been illustrated, and variations of which are described with reference to Figures 18 - 25. Generally, the implantable device may comprise a single large mass or single large protrusion to uniformly apply force to the heart wall and to avoid focused compression of the coronary arteries and cardiac veins. Alternatively, the implantable device may have a relatively small contact area defined by one or a plurality of protrusions selected and positioned to establish localized contact with the heart wall while avoiding contact with and compression of the coronary arteries and cardiac veins.

In Figures 18A and 18B, the implantable device 3010 includes a base 2016 defining a wall 2014 and an interior 2012. A single circular protrusion 2018 extends from the base 2016, which may be in fluid communication therewith. The base 2016 and/or the protrusion 2018 may be expanded to the desired size and shape. The base 2016 may include a securement as described hereinafter, such as a tissue in-growth promoting surface 2017.

In Figures 19A and 19B, the implantable device 3110 is similar to device 3010 described above except that a plurality (e.g., two, three or more) of circular protrusions 2018 extend from the base 2016. This embodiment illustrates that any suitable number of protrusion(s) 2018 may be utilized.

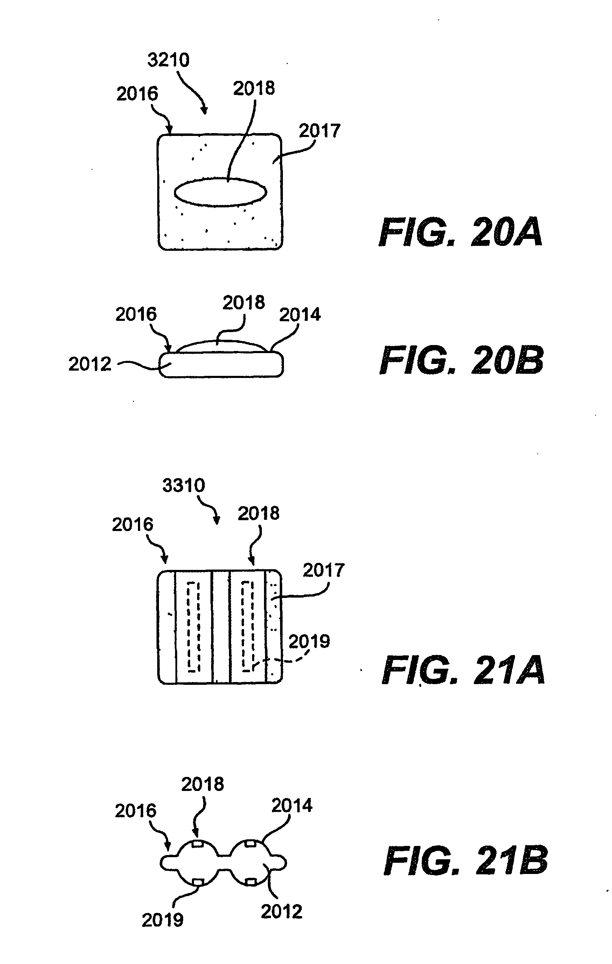

In Figures 20A and 20B, the implantable device 3210 is similar to device 3010 except that a single oblong protrusion 2018 extends from the base 2016. This embodiment illustrates that the protrusion(s) 2018 may assume a wide variety of geometries, including circular and non-circular geometries.

In Figures 21A and 21B, the implantable device 3310 the implantable device 3210 is similar to device 3010 except that one or more elongate protrusions 2018 are integrally formed with and extend from both sides of the base 2016. In addition, reinforcement strips 2019 may be disposed at the apex of the protrusions 2018 to enhance rigidity thereof. This embodiment illustrates that the protrusions 2018 may be integrally formed with the base 2016, and/or may extend from both sides of the base 2016, and/or may be selectively reinforced.

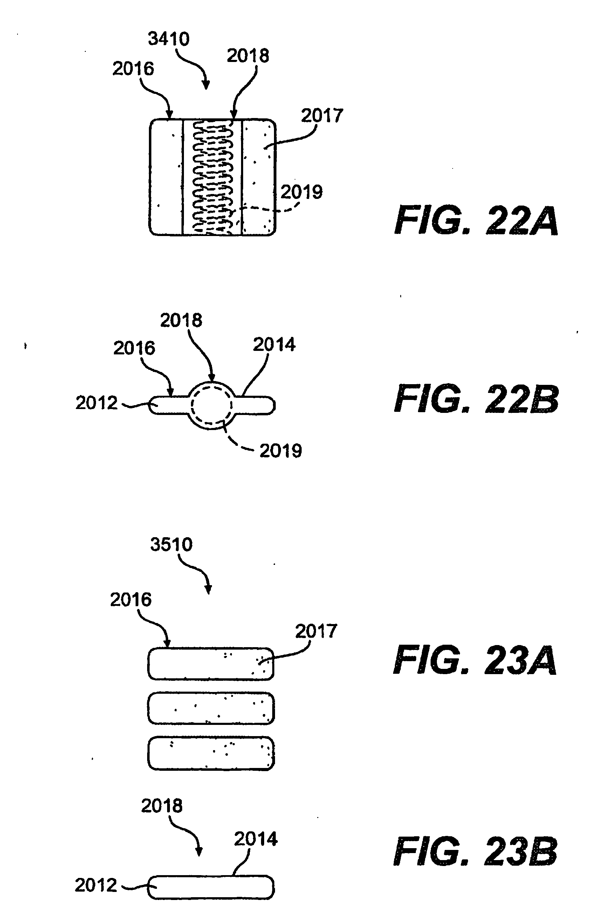

In Figures 22A and 22B, the implantable device 3410 is similar to device 3310 except that the protrusion 2018 includes a reinforcement structure 2019 (e.g., 2-dimensional or 3-dimensional coil or stent) disposed in the interior 2012 thereof to enhance the hoop strength of the protrusion 2018. The reinforcement structure 2019 disposed in the elongate protrusion 18 illustrates that the hoop strength or holding power of the protrusion(s) 2018 may be increased by mechanical means.

In Figures 23A and 23B, the implantable device 3510 is similar to device'3010 except that the device 3510 comprises one or more discrete protrusions 2018. This embodiment illustrates that the implantable device 3510 may comprise one or more individual and separate protrusions 2018 used collectively, which may not define a discrete base portion and a discrete protrusion portion.

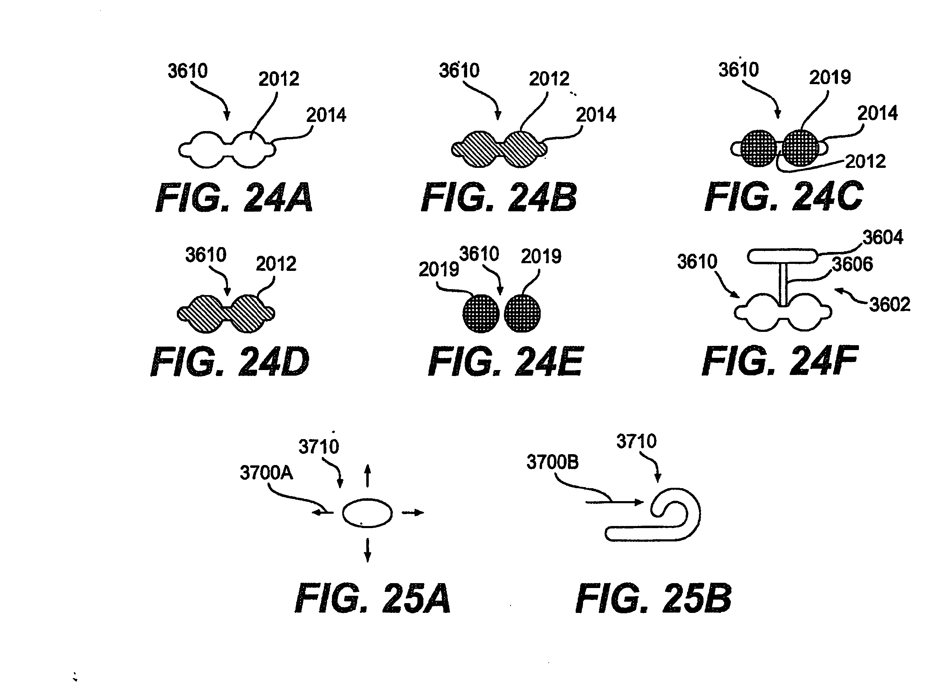

Each implantable device described herein may be expanded or filled by different materials and/or structures, each of which may dictate a different construction of the device as illustrated by the following discussion with reference to Figures 24A - 24F which schematically illustrate different embodiments of an implantable device 3610. The implantable device 3610 may include an interior 2012 defined by wall 2014, wherein the interior 2012 is filled by a fluid as shown in Figure 24A. The fluid may remain a liquid (e.g., saline) or a gas (e.g., carbon dioxide) as shown in Figure 24A, or may comprise or cure into a solid or semi-solid (e.g., gel, expandable foam, sponge, PVA, collagen) as shown in Figure 24B. In addition or in the alternative, a mechanical structure 2019 such as a stent or coil may be placed in the interior 2012 as shown in Figure 24C. To the extent that the wall 2014 is not necessary to contain the solid filler material, the device 3610 may have dissolvable walls or may not have walls at all as shown in Figure 24D. Similarly, to the extent a mechanical structure 2019 such as a stent or coil is used, the device 3610 may not require walls as shown in Figure 24E, and the device 3610 may simply comprise the mechanical structure 2019 itself.

In addition, each of the implantable devices may include a means to secure itself to the heart H wall and/or other surrounding tissue. The securement may comprise tines, screws, sutures, or other structural anchors, and/or the securement may comprise a material (e.g., Dacron fabric) that promotes tissue in-growth. The securement may be remotely activated. For example, the securement may comprise curled wires disposed on either side of the implantable device, wherein the wires curl into the heart wall as they are advanced out of a catheter lumen. The securement may selectively anchor to some tissue while remaining free of other tissue. For example, the securement may anchor to the epicardium and/or myocardium, while remaining free of the pericardium. It has been observed that the epicardium is a relatively tough tissue, thus providing a good anatomical structure to secure the implantable device.

In the embodiments described with reference to Figures 18 - 25, the securement is shown as a tissue in-growth promoting surface on the bottom, and a smooth surface on the top, thus establishing, for example, a secure connection to the epicardium while remaining free of the pericardium. In the embodiment shown in Figure 24F, the device 3610 includes an transmyocardial securement 3602 having an intra-chamber anchor pad 3604 and a connection member 3606. The intra-chamber pad 3604 resides within a chamber (e.g., left ventricle LV) of the heart H, and the connection member 3606 extends through the heart wall (endocardium, myocardium and epicardium) to the implantable device 3610 disposed outside the heart wall. The transmyocardial securement 3602 is particularly suited for the transventricular approach described previously.

Each implantable device described herein may be expandable between a relatively small delivery configuration and a relatively large deployed configuration. The smaller delivery configuration permits the device to be low profile to facilitate advancement through catheter lumens in the various transluminal approaches described herein. For example, the implantable device 3710 may be expanded radially as shown by arrows 3700A in Figure 25A, or unfurled as shown by arrow 3700B in Figure 25B. Radial expansion may be appropriate when the device 3710 is constructed of highly elastic materials (e.g., silicone rubber, latex, elastomeric polymers, etc.) and unfurling may be appropriate when the device 3710 is constructed of relatively inelastic materials (e.g., PET, HDPE, PTFE, SST, Nitinol, etc.).

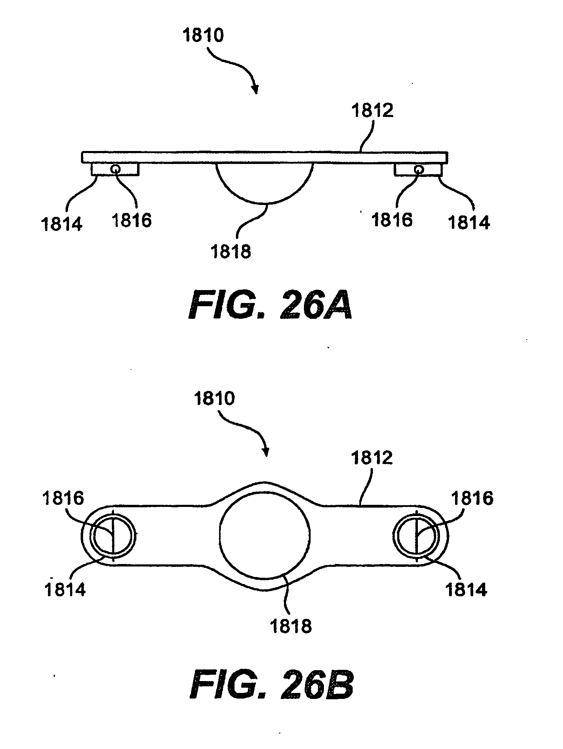

In Figures 26A and 26B, side and bottom views, respectively, are shown of implantable device 1810. Implantable device 1810 includes a base 1812 which may comprise, for example, a flexible polymer sheet having resistance to elongation. Two or more suction cups 1814 are connected to opposite ends of the base 1812. The, suction cups 1814 have an open bottom portion, with the top portion thereof sealing connected to the base 1812. One or more pins 1816 extend through and across each of the suction cups 1814. The pins 1816 may be inserted and locked in holes defined in the walls of the suction cups 1814. A inflatable and deflatable balloon 1818 is connected to and extends from the bottom of the base 1812. The balloon 1818 may be filled with a variety of materials as described previously.

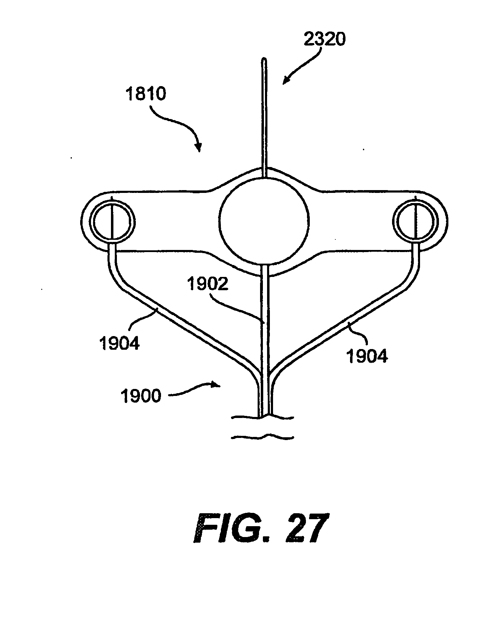

In Figure 27, a bottom view of a delivery catheter 1900 connected to the implantable device 1810 is shown. Delivery catheter 1900 includes an inflation tube 1902 releasably connected to and in fluid communication with the balloon 1818. Inflation tube 1902 includes an inflation lumen extending therethrough, and may include a guide wire lumen for advancement over guide wire 2320 as shown and described with reference to Figures 31A and 31B. The proximal end (not shown) of the inflation tube 1902 may be connected to an inflation device to selectively inflate and deflate the balloon 1818. Delivery catheter 1900 also includes vacuum tubes 1904 releasably connected to and in fluid communication with each of the suction cups 1814. The proximal ends (not shown) of the vacuum tubes 1904 may be connected to an vacuum source to selectively apply suction to the suction cups 1814. The pins 1816 are releasably connected to push/pull wires (not shown) extending through the vacuum tubes 1904 such that the pins may be remotely and selectively advanced and retracted by manipulating the proximal ands of the push/pull wires.

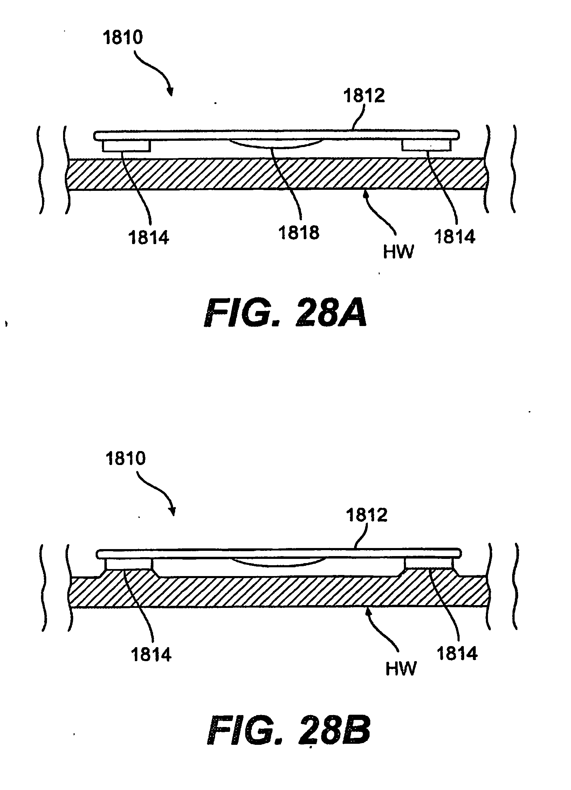

In Figures 28A - 28D, an example of a method of deploying the implantable device 1810 is schematically shown. The implantable device 1810 may be positioned adjacent the heart wall HW (e.g., between the epicardium and pericardium) as shown in Figure 28A, using delivery catheter 1900 (not shown) advanced over guide wire 2320 (shown in Figures 31A and 31B), by a transthoracic approach, for example. The balloon 1818 of implantable device 1810 may be positioned adjacent the MV or a specific part thereof (e.g., annulus AN or papillary, muscles PM) as confirmed using by echocardiography, with the suction cups 1814 avoiding coronary vasculature as confirmed by radiography. For example, the balloon 1818 may be positioned adjacent the annulus and/or posterior papillary muscle PPM, with the suction cups 1814 disposed on opposite sides of the second and third obtuse marginals, such that the device 1810 is inferior of the circumflex artery CFX and straddles the second and third obtuse marginals.

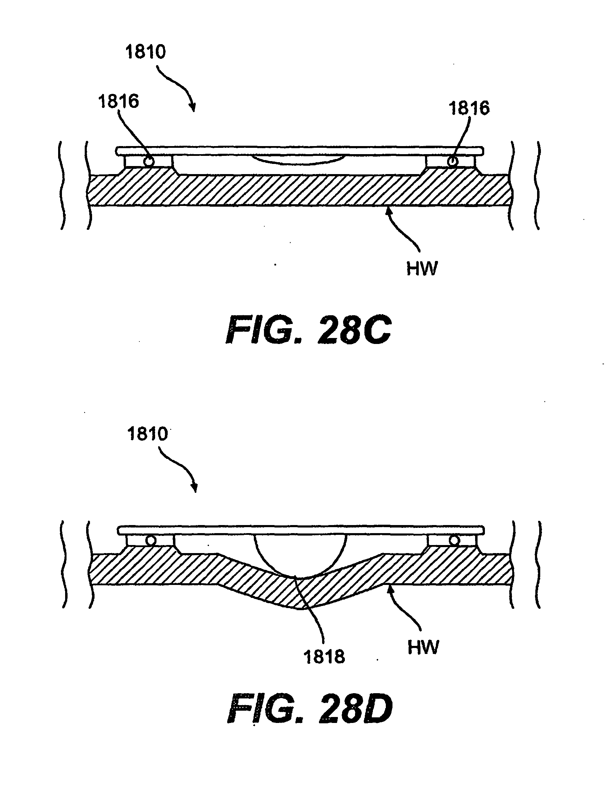

Suction is applied to the suction cups 1814 by vacuum tubes 1904 (not shown), causing a portion of the heart wall HW to be displaced into the interior of each suction cup 1814 as shown in Figure 28B. Pins 1816 may then be advanced through the vacuum tubes 1904 and into each of the suction cups 1814 by remotely pushing on the push/pull wires, thus causing the pins 1816 to pierce the portion of the heart wall HW displaced into the interior of the suction cups 1814 as shown in Figure 28C. The vacuum source may then be deactivated to release the vacuum applied to the suction cups 1814 via vacuum tubes 1904. Because the epicardium of the heart wall HW is a relatively tough tissue, the pins 1816 provide a secure connection to the heart wall HW. As an alternative, the pericardium may be suctioned into the suction cups 1814 such that the pins 1816 pierce the pericardium as well. The balloon 1818 may then be inflated as shown in Figure 28D, and the desired acute effect may be confirmed by echocardiography. The catheter 1900 may then be disconnected from the implantable, device 1810, leaving the balloon 1818 inflated and the pins 1816 secured to the heart wall HW in suction cups 1814.

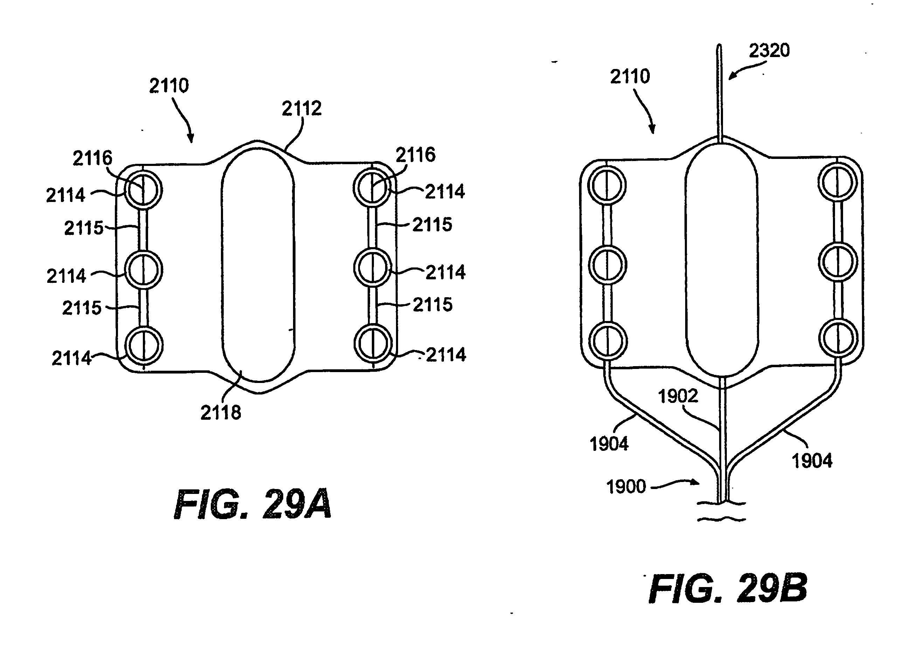

In Figure 29A, a bottom view is shown of an alternative implantable device 2110, which may be similar in design and substantially the same in use as implantable device 1810 described previously. In this alternative embodiment, implantable device 2110 includes a base 2110 which may comprise, for example, a flexible polymer sheet having resistance to elongation. Two series of three suction cups 2114 each are uniformly distributed along and connected to opposite sides of the base 2112, and are interconnected by tubes 2115. A pin 2116 extends through and across each series of the suction cups 2114 and tubes 2115. A inflatable and deflatable balloon 2118 is connected to and extends from the bottom of the base 2112, and may be filled with a variety of materials as described previously.. As compared to the implantable device 1810 described with reference to Figures 26A and 26B, the implantable device 2110 illustrated in Figure 29A utilizes a balloon 2118 having a larger surface area and different geometry, and more suction cups 2114 interconnected by tubes 2115. As shown in Figure 29B, delivery catheter 1900 may be connected to implantable device 2110 in a similar manner as the connection to implantable device 1810 described previously. Further, the steps of deploying implantable device 2110 may be the same as described previously for implantable device 1810.

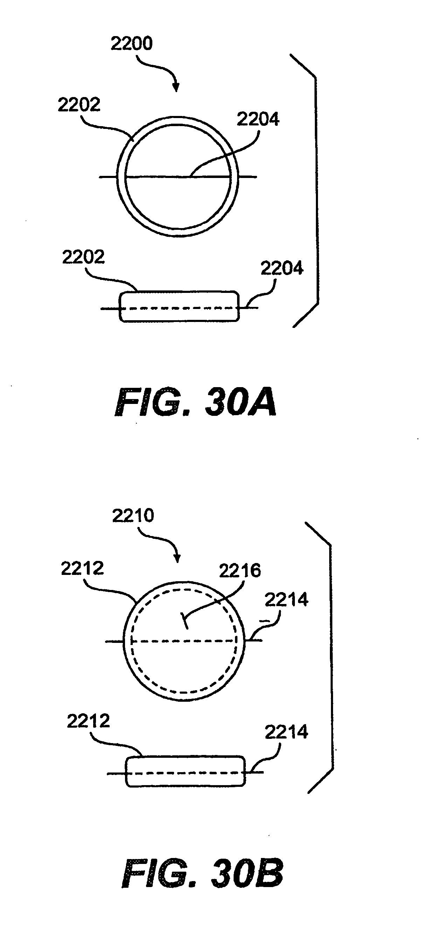

With reference to Figures 30A - 30C, various design alternatives for the suction cups 1814/2114 are shown as top views and side views. In Figure 30A, the suction cup 2200 includes a circular wall portion 2202 defining an interior with an open bottom and top. A pin 2204 extends through holes in the wall 2202 as well as the interior defined by circular wall 2202. With an open bottom and top, suction applied to the cup 2200 pulls both the heart wall (at least the epicardium) and the pericardium into the interior allowing the pin 2204 to pierce through both tissue layers.

In Figure 30B, the suction cup 2210 includes a circular wall portion 2212 defining an interior. A cap 2216 covers the top portion of the wall 2212 to define a closed top portion and an open bottom potion of the cup 2210. A pin 2214 extends through holes in the wall 2212 as well as the interior defined by circular wall 2212. With an open bottom and a closed top, suction applied to the cup 2210 pulls the heart wall (at least the epicardium) into the interior while the cover 2216 prevents the pericardium from entering, thus allowing the pin 2214 to pierce through the heart wall but not the pericardium.

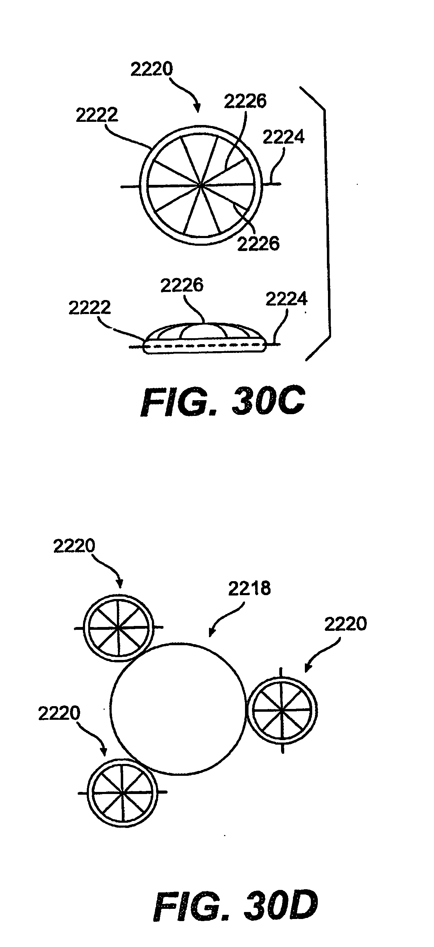

In Figure 30C, the suction cup 2220 includes a circular wall portion 2222 defining an interior. A series of crossing wires 2226 cover the top portion of the wall 2222 to define a screened top portion and an open bottom potion of the cup 2220. The wall 2222 may be formed of a tubular structure with a highly elastic wire (e.g., NiTi) running therethrough, and the wires 2226 may be formed of a highly elastic material (e.g., NiTi) such that the entire cup 2220 may be collapsed into a delivery configuration small enough to fit into a delivery catheter and subsequently deployed into an expanded configuration as shown. A pin 2224 extends through holes in the wall 2222 as well as the interior defined by the wall 2222. With an open bottom and a screened top, suction applied to the cup 2220 pulls the heart wall (at least the epicardium) into the interior. Depending on the density of wires 2226 and the amount of suction applied, the pericardium may be selectively pulled into the interior, thus allowing the pin 2224 to pierce through the heart wall and optionally the pericardium.

With reference to Figure.30D, an alternative implantation arrangement is shown. In this embodiment, three or more suction cups 2220 are attached to the heart wall and pericardium to isolate and hold the balloon 2218 therebetween. By connecting to both the epicardium and the heart wall in three or more locations, the balloon 2218 is constrained by the heart wall, the epicardium and the suction cup anchors 2220. This arrangement eliminates the need to interconnect the balloon 2218 and suction cups 2220 (e.g., by a base structure), and permits the suction cups and balloon to be separately delivered in a smaller profile enabling transluminal delivery through a catheter.

From the foregoing, it will be apparent to those skilled in the art that the present invention provides, in exemplary non-limiting embodiments, devices for improving the function of a valve (e.g., mitral valve) by positioning an implantable device outside and adjacent the heart wall such that the device applies an inward force against the heart wall or otherwise deforms the heart wall thus acting on the valve to improve leaflet coaptation. Further, those skilled in the art will recognize that the present invention may be manifested in a variety of forms other than the specific embodiments described and contemplated herein. Accordingly, departures in form and detail may be made without departing from the scope of the present invention as described in the appended claims.

## Claims

1. A device for improving heart valve function, the device comprising:
a first anchoring member (12,112, 212, 612, 712);
a second anchoring member (14, 114, 214, 614, 714);
an interconnecting member (16, 116, 216, 616, 716) connecting the first anchoring member (12, 112, 212, 612, 712) and the second anchoring member (14, 114, 214, 614, 714), wherein the interconnecting member (16, 116, 216, 616, 716) is configured to be selectively adjustable in length,
**characterized in that**
a protrusion (18, 118, 218, 618, 648, 718, 748, 2018) is connected to the interconnecting member (16, 116, 216, 616, 716) between the first and second anchoring elements, wherein the protrusion (18, 118, 218, 618, 648, 718, 748, 2018) comprises an expandable structure,
wherein at least a portion of the device is configured to be positioned in contact with an external surface of a heart wall proximate a valve such that adjustment of the interconnecting member (16, 116, 216, 616, 716) or expansion of the protrusion (18, 118, 218, 618, 648, 718, 748, 2018) produces an inward force on the heart wall thereby altering the valve function.

2. The device of claim 1, wherein the first anchoring member (12, 112, 212, 612, 712) and the second anchoring member (14, 114, 214, 614, 714) are configured to be secured to an epicardial layer of the heart

3. The device of claim 2, wherein the first anchoring member (12, 112, 212, 612, 712) and the second anchoring member (14, 114, 214, 614, 714) are configured to be secured to the epicardial layer while the anchoring members (12, 14, 112, 114, 212, 214,612,614,712,714) remain free from a pericardial layer of the heart.

4. The device of claim 2, wherein at least one of the first anchoring member (12, 112, 212, 612, 712) and the second anchoring member (14, 114, 214, 614, 714) is configured to be secured to the pericardial layer of the heart.

5. The device of claim 1, wherein the expandable protrusion (18, 118, 218, 618, 648, 718, 748, 2018) is configured to be positioned between the first anchoring member (12, 112, 212, 612, 712) and the second anchoring member (14, 114, 214, 614, 714).

6. The device of claim 1 or 5, wherein the interconnecting member (16, 116, 216, 616, 716) is made of a plurality of filaments.

7. The device of claim 6, wherein the plurality of filaments form a braided structure (132, 134, 758).

8. The device of claim 1 or 5, further comprising a covering provided on the interconnecting member (16,116,216,616, 716).

9. The device of claim 8, wherein the covering is biocompatible.

10. The device of claim 8, wherein the covering is configured to promote tissue ingrowth.

11. The device of claim 8, wherein the covering comprises an atraumatic pad (130).

12. The device of claim 5, wherein the protrusion (18, 118, 218, 618, 648, 718, 748, 2018) is one of fixedly and adjustably connected to the interconnecting member (16, 116,216,616,716).

13. The device of claim 5, wherein the expandable protrusion (18, 118, 218, 618, 648, 718, 748, 2018) is configured to be selectively expandable after implantation of the device relative to the heart.

14. The device of claim 5, wherein the protrusion (l8, 118, 218, 618, 648, 718, 748, 2018) comprises an outer surface configured to promote tissue ingrowth.

15. The device of claim 1 or 5, further comprising a tissue penetrating member associated with each of the first anchoring member (12, 112, 212, 612, 712) and the second anchoring member (14, 114, 214, 614, 714).

16. The device of claim 15, wherein the tissue penetrating members comprise selectively actuatable pins.

17. The device of claim 1 or 5, wherein the first anchoring member (12, 112, 212, 612, 712) and the second anchoring member (14, 114, 214,614, 714) are configured to be positioned so as to avoid coronary arteries and coronary veins when the device is implanted relative to the heart.

18. The device of claim 1 or 5, wherein the interconnecting member (16, 116, 216, 616, 716) is configured to be adjustably connected to at least one of the first (12, 112, 212, 612, 712) and second (14, 114, 214, 614, 714) anchoring members.

19. The device of claim 18, wherein the interconnecting member (16, 116, 216, 616, 716) is configured to be selectively lockable relative to the at least one first and second anchoring member (14,114, 214, 614, 714).

20. The device of claim 19, further comprising a pin configured to selectively penetrate the interconnecting member (16, 116, 216, 616, 716) so as to selectively lock the interconnecting member (16, 116, 216, 616, 716) to the at least one first and second anchoring member (14,114, 214, 614; 714).

21. The device of claim 18, wherein the interconnecting member (16, 116, 216, 616, 716) is configured to be adjustably connected to the first anchoring member (12, 112, 212, 612, 712) and to the second anchoring member (14,114,214,614,7t4).

22. The device of claim 18, wherein the interconnecting member (16, 116, 216, 616, 716) is configured to be adjustably connected to one of the first anchoring member (12, 112, 212, 612, 712) and the second anchoring member (14, 114, 214, 614, 714) and fixedly connected to the other of the first anchoring member (12, 112, 212, 612, 712) and the second anchoring member (14,114,214,614, 714).

23. The device of claim 5, wherein the interconnecting member (16, 116, 216, 616, 716) is configured to be adjustably connected to the protrusion (18, 118, 218, 618, 648, 718, 748, 2018) and fixedly connected to the first anchoring member (12, 112, 212, 612, 712) and to the second anchoring member (14, 114, 214, 614, 714).

24. The device of claim 5, wherein the interconnecting member (16, 116, 216, 616, 716) comprises a first interconnecting member (16, 116, 216, 616, 716) connecting the protrusion (18, 118, 218, 618, 648, 718, 748, 2018) to the first anchoring member (12, 112, 212, 612, 712) and a second interconnecting member (16, 116, 216, 616, 716) connecting the protrusion (18, 118, 218, 618, 648, 718, 748, 2018) to the second anchoring member (14, 114, 214, 614, 714).

25. The device of claim 5, wherein at least a portion of the first and second anchoring member (14, 114, 214, 614, 714) is remotely actuatable so as to embed into heart tissue.

26. The device of claim 25, wherein the portion of the first anchoring member (12, 112, 2I2, 612, 712) and the portion of the second anchoring member (14,114, 214, 614, 714) comprise tissue penetrating members.

27. The device of claim 26, wherein the tissue penetrating members are pins.

28. The device of claim 25, wherein the portion of the first anchoring member (12, 112, 212, 612, 712) and the portion of the second anchoring member (14, 114, 214, 614, 714) are configured to be remotely actuable via push-pull member associated with a catheter.

29. The device of any one of claims 1, 5 or 25, wherein the first anchoring member (12, 112, 212, 612, 712) and the second anchoring member (14, 114, 214, 614, 714) define vacuum chambers.

30. The device of claim 1, wherein the interconnecting member (16,116, 216, 616, 716) is flexible.

31. The device of claim 5, wherein the interconnecting member (16, 116, 216, 616, 716) is flexible.

32. The device of claim 31, wherein the device is configured such that the device exerts a force substantially opposite to the inward force by securing the device to the heart wall.

33. The device of claim 31, wherein the device is configured such that the device exerts a force substantially opposite to the inward force against anatomical structure outside the heart wall.

34. The device of claims 1, 5 or 31, wherein the protrusion (18, 118, 218, 618, 648, 718, 748, 2018) comprises an inflatable structure.

35. The device of claim 34, wherein the protrusion (18, 118, 218, 618, 648, 718, 748, 2018) includes a balloon (142,2084, 2118,2218).

36. The device of claims 1,5 or 31, wherein the protrusion (18,118, 218, 618, 648, 718, 748,2018) defines an interior.

37. The device of claim 36, further comprising a coil disposed in the interior of the protrusion.

38. The device of claim 36, further comprising a foam disposed in the interior of the protrusion.

39. The device of claim 36, further comprising a sponge disposed in the interior of the protrusion.

40. The device of claim 36, further comprising a liquid disposed in the interior of the protrusion.

41. The device of claim 40, wherein the liquid is a curable liquid.

42. The device of claim 36, further comprising a mechanical reinforcement member (2019) disposed in the interior of the protrusion.

43. The device of claim 36, further comprising means for selectively adding or removing material from the interior.

44. The device of claim 43, wherein the means is transdermally accessible.

45. The device of claim 31, wherein the protrusion (18, 118, 218, 618, 648, 718, 748, 2018) protrudes from the flexible member.

46. The device of claim 31, wherein the protrusion (18, 118, 218, 618, 648, 718, 748, 2018) is expandable between a relatively small delivery configuration and a relatively large deployed configuration.

47. The device of claim 1 or 5 or 31, wherein the device is configured to be delivered to the heart via a delivery catheter.

48. The device of claim 30 or 31, wherein the device is configured to be delivered to the heart via a delivery catheter and wherein the device is configured to be releasably connected to the delivery catheter.

49. The device of claim 31, wherein the at least one protrusion (18, 118, 218, 618, 648, 718, 748, 2018) is configured to exert an inward force sufficient to draw leaflets of the valve together.

50. The device of claim 31, wherein the anchoring members (12, 14, 112, 114, 212, 214, 612, 614, 712, 714) are configured to secure the device to the heart

51. The device of claim 32 or 33, wherein the force exerted substantially opposite the inward force is substantially equal to the inward force.

## Patentansprüche

1. Vorrichtung zur Verbesserung der Herzklappenfunktion mit
einem ersten Verankerungselement (12, 112, 212, 612, 712),
einem zweiten Verankerungselement (14, 114, 214, 614, 714),
einem Verbindungselement (16, 116, 216, 616, 716), das das erste Verankerungselement (12, 112, 212, 612, 712) mit dem zweiten Verankerungselement (14, 114, 214, 614, 714) verbindet, wobei das Verbindungselement (16, 116, 216, 616, 716) so aufgebaut ist, dass seine Länge selektiv einstellbar ist,
**dadurch gekennzeichnet, dass**
zwischen dem ersten und dem zweiten Verankerungselement eine Ausbuchtung (18, 118, 218, 618, 648, 718, 748, 2018), die eine aufweitbare Struktur aufweist, mit dem Verbindungselement (16, 116, 216, 616, 716) verbunden ist,
wobei zumindest ein Teil der Vorrichtung so aufgebaut ist, dass er in der Nähe einer Klappe so in Kontakt mit einer Außenfläche einer Herzwand angeordnet wird, dass eine Einstellung des Verbindungselements (16, 116, 216, 616, 716) oder eine Aufweitung der Ausbuchtung (18, 118, 218, 618, 648, 718, 748, 2018) eine nach innen gerichtete, auf die Herzwand einwirkende Kraft erzeugt, wodurch die Klappenfunktion verändert wird.

2. Vorrichtung nach Anspruch 1, bei der das erste Verankerungselement (12, 112, 212, 612, 712) und das zweite Verankerungselement (14, 114, 214, 614, 714) so aufgebaut sind, dass sie an einer epikardialen Schicht des Herzens befestigbar sind

3. Vorrichtung nach Anspruch 2, bei der das erste Verankerungselement (12, 112, 212, 612, 712) und das zweite Verankerungselement (14, 114, 214, 614, 714) so aufgebaut sind, dass sie an einer epikardialen Schicht befestigbar sind, wobei die Verankerungselemente (12, 14, 112, 114, 212, 214, 612, 614, 712, 714) von einer perikardialen Schicht des Herzens gelöst bleiben.

4. Vorrichtung nach Anspruch 2, bei der zumindest entweder das erste Verankerungselement (12, 112, 212, 612, 712) oder das zweite Verankerungselement (14, 114, 214, 614, 714) so aufgebaut ist, dass es an der perikardialen Schicht des Herzens befestigbar ist.

5. Vorrichtung nach Anspruch 1, bei der die aufweitbare Ausbuchtung (18, 118, 218, 618, 648, 718, 748, 2018) so aufgebaut ist, dass sie zwischen dem ersten Verankerungselement (12, 112, 212, 612, 712) und dem zweiten Verankerungselement (14, 114, 214, 614, 714) angeordnet ist.

6. Vorrichtung nach Anspruch 1 oder 5, bei der das Verbindungselement (16, 116, 216, 616, 716) aus mehreren Filamenten gefertigt ist.

7. Vorrichtung nach Anspruch 6, bei der die mehreren Filamente eine geflochtene Struktur (132, 134, 758) bilden.

8. Vorrichtung nach Anspruch 1 oder 5, die ferner eine auf dem Verbindungselement (16, 116, 216, 616, 716) vorgesehene Abdeckung aufweist.

9. Vorrichtung nach Anspruch 8, bei der die Abdeckung biokompatibel ist.

10. Vorrichtung nach Anspruch 8, bei der die Abdeckung so konfiguriert ist, dass sie das Einwachsen in Gewebe fördert.

11. Vorrichtung nach Anspruch 8, bei der die Abdeckung ein antitraumatisches Polster aufweist.

12. Vorrichtung nach Anspruch 5, bei der die Ausbuchtung (18, 118, 218, 618, 648, 718, 748, 2018) entweder fest oder einstellbar mit dem Verbindungselement (16, 116, 216, 616, 716) verbunden ist.

13. Vorrichtung nach Anspruch 5, bei der die aufweitbare Ausbuchtung (18, 118, 218, 618, 648, 718, 748, 2018) so aufgebaut ist, dass sie nach der Implantation der Vorrichtung relativ zum Herzen selektiv aufweitbar ist.

14. Vorrichtung nach Anspruch 5, bei der die Ausbuchtung (18, 118, 218, 618, 648, 718, 748, 2018) eine Außenfläche aufweist, die so aufgebaut ist, dass sie ein Einwachsen in Gewebe fördert.

15. Vorrichtung nach Anspruch 1 oder 5, die ferner sowohl ein dem ersten Verankerungselement (12, 112, 212, 612, 712) als auch ein dem zweiten Verankerungselement (14, 114, 214, 614, 714) zugeordnetes Gewebepenetrationselement umfasst.

16. Vorrichtung nach Anspruch 15, bei der die Gewebepenetrationselemente selektiv auslösbare Stifte umfassen.

17. Vorrichtung nach Anspruch 1 oder 5, bei der das erste Verankerungselement (12, 112, 212, 612, 712) und das zweite Verankerungselement (14, 114, 214, 614, 714) so aufgebaut sind, dass sie so angeordnet werden, dass sie Koronararterien und Koronarvenen vermeiden, wenn die Vorrichtung relativ zum Herzen implantiert wird.

18. Vorrichtung nach Anspruch 1 oder 5, bei der das Verbindungselement (16, 116, 216, 616, 716) so aufgebaut ist, dass es einstellbar mit zumindest entweder dem ersten (12, 112, 212, 612, 712) oder dem zweiten Verankerungselement (14, 114, 214, 614, 714) verbunden ist.

19. Vorrichtung nach Anspruch 18, bei der das Verbindungselement (16, 116, 216, 616, 716) so aufgebaut ist, dass es in Bezug auf zumindest entweder das erste oder das zweite Verankerungselement (14, 114, 214, 614, 714) selektiv arretierbar ist.

20. Vorrichtung nach Anspruch 19, die ferner einen Stift umfasst, der so konfiguriert ist, dass er selektiv in das Verbindungselement (16, 116, 216, 616, 716) eindringt, um das Verbindungselement (16, 116, 216, 616, 716) selektiv zumindest entweder am ersten oder am zweiten Verankerungselement (14, 114, 214, 614, 714) zu arretieren.

21. Vorrichtung nach Anspruch 18, bei der das Verbindungselement (16, 116, 216, 616, 716) so aufgebaut ist, dass es einstellbar mit dem ersten Verankerungselement (12, 112, 212, 612, 712) und dem zweiten Verankerungselement (14, 114, 214, 614, 714) verbunden ist.

22. Vorrichtung nach Anspruch 18, bei der das Verbindungselement (16, 116, 216, 616, 716) so aufgebaut ist, dass es mit einem der beiden Verankerungselemente, dem ersten Verankerungselement (12, 112, 212, 612, 712) oder mit dem zweiten Verankerungselement (14, 114, 214, 614, 714) einstellbar und mit anderen der beiden Verankerungselemente, dem ersten Verankerungselement (12, 112, 212, 612, 712) oder dem zweiten Verankerungselement (14, 114, 214, 614, 714), fest verbunden ist.

23. Vorrichtung nach Anspruch 5, bei der das Verbindungselement (16, 116, 216, 616, 716) so aufgebaut ist, dass es mit der Ausbuchtung (18, 118, 218, 618, 648, 718, 748, 2018) einstellbar und mit dem ersten Verankerungselement (12, 112, 212, 612, 712) und dem zweiten Verankerungselement (14, 114, 214, 614, 714) fest verbunden ist.

24. Vorrichtung nach Anspruch 5, bei der das Verbindungselement (16, 116, 216, 616, 716) ein erstes Verbindungselement (16, 116, 216, 616, 716), das die Ausbuchtung (18, 118, 218, 618, 648, 718, 748, 2018) mit dem ersten Verankerungselement (12, 112, 212, 612, 712) verbindet, und ein zweites Verbindungselement (16, 116, 216, 616, 716) umfasst, das die Ausbuchtung (18, 118, 218, 618, 648, 718, 748, 2018) mit dem zweiten Verankerungselement (14, 114, 214, 614, 714) verbindet.

25. Vorrichtung nach Anspruch 5, bei der zumindest ein Abschnitt des ersten und des zweiten Verankerungselements (14, 114, 214, 614, 714) zur Einbettung in Herzgewebe fernauslösbar ist.

26. Vorrichtung nach Anspruch 25, bei der der Abschnitt des ersten Verankerungselements (12, 112, 212, 612, 712) und der Abschnitt des zweiten Verankerungselements (14, 114, 214, 614, 714) Gewebepenetrationselemente aufweisen.

27. Vorrichtung nach Anspruch 26, bei der die Gewebepenetrationselemente Stifte sind.

28. Vorrichtung nach Anspruch 25, bei der der Abschnitt des ersten Verankerungselements (12, 112, 212, 612, 712) und der Abschnitt des zweiten Verankerungselements (14, 114, 214, 614, 714) so aufgebaut sind, dass sie über ein zu einem Katheter gehöriges Druck- und Zugelement fernauslösbar sind.

29. Vorrichtung nach einem der Ansprüche 1, 5 oder 25, bei der das erste Verankerungselement (12, 112, 212, 612, 712) und das zweite Verankerungselement (14, 114, 214, 614, 714) Vakuumkammern definieren.

30. Vorrichtung nach Anspruch 1, bei der das Verbindungselement (16, 116, 216, 616, 716) flexibel ist.

31. Vorrichtung nach Anspruch 5, bei der das Verbindungselement (16, 116, 216, 616, 716) flexibel ist.

32. Vorrichtung nach Anspruch 31, die so aufgebaut ist, dass sie durch ihre Befestigung an der Herzwand eine der nach innen gerichteten Kraft im Wesentlichen entgegengesetzte Kraft aufbringt.

33. Vorrichtung nach Anspruch 31, die so aufgebaut ist, dass sie eine der nach innen gerichteten Kraft im Wesentlichen entgegengesetzte Kraft auf die anatomische Struktur auf der Außenseite der Herzwand aufbringt.

34. Vorrichtung nach Anspruch 1, 5 oder 31, bei der die Ausbuchtung (18, 118, 218, 618, 648, 718, 748, 2018) eine aufblasbare Struktur aufweist.

35. Vorrichtung nach Anspruch 34, bei der die Ausbuchtung (18, 118, 218, 618, 648, 718, 748, 2018) einen Ballon (142, 2084, 2118, 2218) umfasst.

36. Vorrichtung nach Anspruch 1, 5 oder 31, bei der die Ausbuchtung (18, 118, 218, 618, 648, 718, 748, 2018) einen Innenraum definiert.

37. Vorrichtung nach Anspruch 36, die ferner eine im Inneren der Ausbuchtung angeordnete Spule aufweist.

38. Vorrichtung nach Anspruch 36, die ferner im Inneren der Ausbuchtung angeordneten Schaum aufweist.

39. Vorrichtung nach Anspruch 36, die ferner einen im Inneren der Ausbuchtung angeordneten Schwamm aufweist.

40. Vorrichtung nach Anspruch 36, die ferner eine im Inneren der Ausbuchtung angeordnete Flüssigkeit aufweist.

41. Vorrichtung nach Anspruch 40, bei der die Flüssigkeit eine aushärtende Flüssigkeit ist.

42. Vorrichtung nach Anspruch 36, die ferner ein im Inneren der Ausbuchtung angeordnetes mechanisches Verstärkungselement (2019) aufweist.

43. Vorrichtung nach Anspruch 36, die ferner eine Einrichtung zum selektiven Hinzufügen oder Entfernen eines Materials aus dem Inneren aufweist.

44. Vorrichtung nach Anspruch 43, bei der die Einrichtung transdermal zugänglich ist.

45. Vorrichtung nach Anspruch 31, bei der die Ausbuchtung (18, 118, 218, 618, 648, 718, 748, 2018) aus dem flexiblen Element ragt.

46. Vorrichtung nach Anspruch 31, bei der die Ausbuchtung (18, 118, 218, 618, 648, 718, 748, 2018) von einer relativ kleinen Lieferkonfiguration zu einer relativ großen geöffneten Konfiguration aufweitbar ist.

47. Vorrichtung nach Anspruch 1, 5 oder 31, die so aufgebaut ist, dass sie über einen Abgabekatheter zum Herzen befördert wird.

48. Vorrichtung nach Anspruch 30 oder 31, die so aufgebaut ist, dass sie über einen Abgabekatheter zum Herzen befördert wird, wobei die Vorrichtung so aufgebaut ist, dass sie lösbar mit dem Abgabekatheter verbunden ist.

49. Vorrichtung nach Anspruch 31, bei der die zumindest eine Ausbuchtung (18, 118, 218, 618, 648, 718, 748, 2018) so aufgebaut ist, dass sie eine nach innen gerichtete Kraft aufbringt, die ausreicht, um die Fiederblätter der Klappe zusammenzuziehen.

50. Vorrichtung nach Anspruch 31, bei der die Verankerungselemente (12, 14, 112, 114, 212, 214, 612, 614, 712, 714) so aufgebaut sind, dass sie die Vorrichtung am Herzen befestigen.

51. Vorrichtung nach Anspruch 32 oder 33, bei der die im Wesentlichen entgegengesetzt zu der nach innen gerichteten Kraft aufgebrachte Kraft im Wesentlichen mit der nach innen gerichteten Kraft übereinstimmt.

## Revendications

1. Dispositif pour améliorer la fonction d'une valvule cardiaque, le dispositif comprenant :
un premier élément d'ancrage (12, 112, 212, 612, 712) ;
un second élément d'ancrage (14, 114, 214, 614, 714) ;
un élément d'interconnexion (16, 116, 216, 616, 716) reliant le premier élément d'ancrage (12, 112, 212, 612, 712) et le second élément d'ancrage (14, 114, 214, 614, 714), dans lequel l'élément d'interconnexion (16, 116, 216, 616, 716) est configuré pour être ajustable sélectivement en longueur,
**caractérisé en ce que**
une protubérance (18, 118, 218, 618, 648, 718, 748, 2018) est reliée à l'élément d'interconnexion (16, 116, 216, 616, 716) entre le premier et le second élément d'ancrage, la protubérance (18, 118, 218, 618, 648, 718, 748, 2018) comprenant une structure extensible,
dans lequel au moins une partie du dispositif est configurée pour être positionnée en contact avec une surface externe d'une paroi du coeur à proximité d'une valvule de sorte que l'ajustement de l'élément d'interconnexion (16, 116, 216, 616, 716) ou l'expansion de la protubérance (18, 118, 218, 618, 648, 718, 748, 2018) produise une force interne sur la paroi du coeur, altérant ainsi la fonction de valvule.

2. Dispositif selon la revendication 1, dans lequel le premier élément d'ancrage (12, 112, 212, 612, 712) et le second élément d'ancrage (14, 114, 214, 614, 714) sont configurés pour être fixés à une couche épicardique du coeur.

3. Dispositif selon la revendication 2, dans lequel le premier élément d'ancrage (12, 112, 212, 612, 712) et le second élément d'ancrage (14, 114, 214, 614, 714) sont configurés pour être fixés à la couche épicardique pendant que les éléments d'ancrage (12, 14, 114, 114, 212, 214, 612, 614, 712, 714) restent libres d'une couche péricardique du coeur.

4. Dispositif selon la revendication 2, dans lequel au moins l'un du premier élément d'ancrage (12, 112, 212, 612, 712) et du second élément d'ancrage (14, 114, 214, 614, 714) est configuré pour être fixé à la couche péricardique du coeur.

5. Dispositif selon la revendication 1, dans lequel la protubérance extensible (18, 118, 218, 618, 648, 718, 748, 2018) est configurée pour être positionnée entre le premier élément d'ancrage (12, 112, 212, 612, 712) et le second élément d'ancrage (14, 114, 214, 614, 714).

6. Dispositif selon la revendication 1 ou 5, dans lequel l'élément d'interconnexion (16, 116, 216, 616, 716) est constitué d'une pluralité de filaments.

7. Dispositif selon la revendication 6, dans lequel la pluralité de filaments forme une structure tressée (132, 134, 758).

8. Dispositif selon la revendication 1 ou 5, comprenant en outre un revêtement fourni sur l'élément d'interconnexion (16, 116, 216, 616, 716).

9. Dispositif selon la revendication 8, dans lequel le revêtement est biocompatible.

10. Dispositif selon la revendication 8, dans lequel le revêtement est configuré pour promouvoir la croissance tissulaire.

11. Dispositif selon la revendication 8, dans lequel le revêtement comprend un coussin atraumatique (130).

12. Dispositif selon la revendication 5, dans lequel la protubérance (18, 118, 218, 618, 648, 718, 748, 2018) est reliée de manière fixe ou ajustable à l'élément d'interconnexion (16, 116, 216, 616, 716).

13. Dispositif selon la revendication 5, dans lequel la protubérance extensible (18, 118, 218, 618, 648, 718, 748, 2018) est configurée pour être extensible sélectivement après implantation du dispositif relativement au coeur.

14. Dispositif selon la revendication 5, dans lequel la protubérance (18, 118, 218, 618, 648, 718, 748, 2018) comprend une surface externe configurée pour favoriser la croissance tissulaire.

15. Dispositif selon la revendication 1 ou 5, comprenant en outre un élément de pénétration du tissu associé à chacun du premier élément d'ancrage (12, 112, 212, 612, 712) et du second élément d'ancrage (14, 114, 214, 614, 714).

16. Dispositif selon la revendication 15, dans lequel les éléments de pénétration du tissu comprennent des broches actionnables sélectivement.

17. Dispositif selon la revendication 1 ou 5, dans lequel le premier élément d'ancrage (12, 112, 212, 612, 712) et le second élément d'ancrage (14, 114, 214, 614, 714) sont configurés pour être positionnés de manière à éviter les artères coronaires et les veines coronaires lorsque le dispositif est implanté relativement au coeur.

18. Dispositif selon la revendication 1 ou 5, dans lequel l'élément d'interconnexion (16, 116, 216, 616, 716) est configuré pour être relié de manière ajustable à au moins l'un du premier élément d'ancrage (12, 112, 212, 612, 712) et du second élément d'ancrage (14, 114, 214, 614, 714).

19. Dispositif selon la revendication 18, dans lequel l'élément d'interconnexion (16, 116, 216, 616, 716) est configuré pour pouvoir être verrouillé de manière ajustable à au moins un premier et second élément d'ancrage (14, 114, 214, 614, 714).

20. Dispositif selon la revendication 19, comprenant en outre une broche configurée pour pénétrer sélectivement l'élément d'interconnexion (16, 116, 216, 616, 716) de manière à verrouiller sélectivement l'élément d'interconnexion (16, 116, 216, 616, 716) à au moins un premier et second élément d'ancrage (14, 114, 214, 614, 714).

21. Dispositif selon la revendication 18, dans lequel l'élément d'interconnexion (16, 116, 216, 616, 716) est configuré pour être relié de manière ajustable au premier élément d'ancrage (12, 112, 212, 612, 712) et au second élément d'ancrage (14, 114, 214, 614, 714).

22. Dispositif selon la revendication 18, dans lequel l'élément d'interconnexion (16, 116, 216, 616, 716) est configuré pour être relié de manière ajustable au premier élément d'ancrage (12, 112, 212, 612, 712) ou au second élément d'ancrage (14, 114, 214, 614, 714) et pour être relié de manière fixe à l'autre du premier élément d'ancrage (12, 112, 212, 612, 712) ou du second élément d'ancrage (14, 114, 214, 614, 714).

23. Dispositif selon la revendication 5, dans lequel l'élément d'interconnexion (16, 116, 216, 616, 716) est configuré pour être relié de manière ajustable à la protubérance (18, 118, 218, 618, 648, 718, 748, 2018) et pour être relié de manière fixe au premier élément d'ancrage (12, 112, 212, 612, 712) et au second élément d'ancrage (14, 114, 214, 614, 714).

24. Dispositif selon la revendication 5, dans lequel l'élément d'interconnexion (16, 116, 216, 616, 716) comprend un premier élément d'interconnexion (16, 116, 216, 616, 716) reliant la protubérance (18, 118, 218, 618, 648, 718, 748, 2018) au premier élément d'ancrage (12, 112, 212, 612, 712) et un second élément d'interconnexion (16, 116, 216, 616, 716) reliant la protubérance (18, 118, 218, 618, 648, 718, 748, 2018) au second élément d'ancrage (14, 114, 214, 614, 714).

25. Dispositif selon la revendication 5, dans lequel au moins une partie du premier élément et du second élément d'ancrage (14, 114, 214, 614, 714) est actionnable à distance de manière à s'inclure dans le tissu du coeur.

26. Dispositif selon la revendication 25, dans lequel la partie du premier élément d'ancrage (12, 112, 212, 612, 712) et la partie du second élément d'ancrage (14, 114, 214, 614, 714) comprennent des éléments de pénétration du tissu.

27. Dispositif selon la revendication 26, dans lequel les éléments de pénétration du tissu sont des broches.

28. Dispositif selon la revendication 25, dans lequel la partie du premier élément d'ancrage (12, 112, 212, 612, 712) et la partie du second élément d'ancrage (14, 114, 214, 614, 714) sont configurées pour être actionnables à distance par un élément push-pull associé à un cathéter.

29. Dispositif selon l'une quelconque des revendications 1, 5 ou 25, dans lequel le premier élément d'ancrage (12, 112, 212, 612, 712) et le second élément d'ancrage (14, 114, 214, 614, 714) définissent des chambres de vide.

30. Dispositif selon la revendication 1, dans lequel l'élément d'interconnexion (16, 116, 216, 616, 716) est flexible.

31. Dispositif selon la revendication 5, dans lequel l'élément d'interconnexion (16, 116, 216, 616, 716) est flexible.

32. Dispositif selon la revendication 31, dans lequel le dispositif est configuré de telle sorte que le dispositif exerce une force sensiblement opposée à la force vers l'intérieur par fixation du dispositif à la paroi du coeur.

33. Dispositif selon la revendication 31, dans lequel le dispositif est configuré de telle sorte que le dispositif exerce une force sensiblement opposée à la force vers l'intérieur contre une structure anatomique à l'extérieur de la paroi du coeur.

34. Dispositif selon les revendications 1, 5 ou 31, dans lequel la protubérance (18, 118, 218, 618, 648, 718, 748, 2018) comprend une structure gonflable.

35. Dispositif selon la revendication 34, dans lequel la protubérance (18, 118, 218, 618, 648, 718, 748, 2018) inclut un ballonnet (142, 2084, 2118, 2218).

36. Dispositif selon les revendications 1, 5 ou 31, dans lequel la protubérance (18, 118, 218, 618, 648, 718, 748, 2018) définit un intérieur.

37. Dispositif selon la revendication 36, comprenant en outre une bobine disposée dans l'intérieur de la protubérance.

38. Dispositif selon la revendication 36, comprenant en outre une mousse disposée dans l'intérieur de la protubérance.

39. Dispositif selon la revendication 36, comprenant en outre une éponge disposée dans l'intérieur de la protubérance.

40. Dispositif selon la revendication 36, comprenant en outre un liquide disposé dans l'intérieur de la protubérance.

41. Dispositif selon la revendication 40, dans lequel le liquide est un liquide durcissable.

42. Dispositif selon la revendication 36, comprenant en outre un élément de renforcement mécanique (2019) disposé dans l'intérieur de la protubérance.

43. Dispositif selon la revendication 36, comprenant en outre un moyen pour ajouter ou retirer sélectivement du matériau depuis l'intérieur.

44. Dispositif selon la revendication 43, dans lequel le moyen est accessible par voie transdermique.

45. Dispositif selon la revendication 31, dans lequel la protubérance (18, 118, 218, 618, 648, 718, 748, 2018) dépasse de l'élément flexible.

46. Dispositif selon la revendication 31, dans lequel la protubérance (18, 118, 218, 618, 648, 718, 748, 2018) est extensible entre une configuration de mise en place relativement petite et une configuration déployée relativement grande.

47. Dispositif selon la revendication 1 ou 5 ou 31, dans lequel le dispositif est configuré pour être mis en place dans le coeur *via* un cathéter de mise en place.

48. Dispositif selon la revendication 30 ou 31, dans lequel le dispositif est configuré pour être mis en place dans le coeur *via* un cathéter de mise en place et dans lequel le dispositif est configuré pour être relié de manière libérable au cathéter de mise en place.

49. Dispositif selon la revendication 31, dans lequel la au moins une protubérance (18, 118, 218, 618, 648, 718, 748, 2018) est configurée pour exercer une force vers l'intérieur suffisante pour rapprocher les valves de la valvule.

50. Dispositif selon la revendication 31, dans lequel les éléments d'ancrage (12, 14, 112, 114, 212, 214, 612, 614, 712, 714) sont configurés pour fixer le dispositif au coeur.

51. Dispositif selon la revendication 32 ou 33, dans lequel la force exercée sensiblement opposée à la force vers l'intérieur est sensiblement égale à la force vers l'intérieur.
